# EUROPEAN PATENT SPECIFICATION

(11) **EP 1 920 795 B1**
(45) Date of publication and mention of the grant of the patent: **24.02.2016**
(21) Application number: 06782837.6
(22) Date of filing: 14.08.2006
(51) Int. Cl.: A61M 25/01, A61M 25/09, A61M 25/00

(54) **MEDICAL DEVICE INSERTED IN BODY CAVITY**
IN EINE KÖRPERHÖHLE EINGEFÜHRTES MEDIZINPRODUKT
DISPOSITIF MÉDICAL INSÉRÉ DANS UNE CAVITÉ CORPORELLE

(30) Priority: 29.08.2005 JP 2005247929
(43) Date of publication of application: 14.05.2008
(73) Proprietor: Japan Lifeline Co., Ltd., Tokyo 140-0002 (JP)
(72) Inventor: YOSHIZAKI, Masato c/o Japan Lifeline Co., Ltd., Tokyo 115-0051 (JP); KAWABATA, Takashi c/o Japan Lifeline Co., Ltd., Tokyo 115-0051 (JP)
(74) Representative: Gille Hrabal
(86) International application number: PCT/JP2006/316279
(87) International publication number: WO 2007/026555

(56) References cited:
- EP-A1- 0 778 043
- EP-A2- 0 745 407
- WO-A1-99/23958
- JP-A- 03 141 958
- JP-A- 03 168 158
- JP-A- 08 252 318
- JP-A- 2003 320 034
- US-A- 5 295 493
- US-A1- 2005 113 862
- US-B1- 6 558 368

## Description

### TECHNICAL FIELD

The present invention relates to a medical apparatus for insertion into a body cavity and, more particularly, to a medical apparatus for insertion into a body cavity which can spontaneously control the direction thereof and deliver the distal tip to a predesignated object portion in a living organ accurately and easily by pushing the apparatus into a body cavity.

### BACKGROUND ART

A stylet made of an elastic linear body is used for insertion of a flexible tubular medical apparatus such as a pacing lead for artificially giving stimuli to the muscle of heart or sensing the intracardiac potential, a catheter for injecting high calorie infusion into a middle cardiac vein and an endotracheal tube inserted into the trachea to surely maintain the air way. For treatments such as the percutaneous transluminal angioplasty and stenting and examinations such as angiocardiography, it is conducted that a guide wire made of an elastic linear body is inserted into a blood vessel and, then, a catheter is inserted to the predesignated position in the blood vessel along the guide wire.

Since the apparatus for insertion into the body cavity such as the stylet and the guide wire is pushed into a blood vessel having curves in complicated shapes and branches, excellent skill is required to deliver the distal tip to the predesignated object portion without causing damages to the blood vessel and without straying into portions different from the object portion. There is the possibility that a mistake in the operation causes problems such as cardiac perforation, cardiac tamponade, pneumothorax and hemothorax. As the stylet, a linear stylet having the entirely linear shape, a J-shaped stylet having a distal tip portion bent in the J-shape and a steerable stylet which can provide shapes to the distal tip portion by manipulation by hands are used at present. The linear stylet has a drawback in that selecting the blood vessel correctly at branches is difficult and delivering the stylet to the object portion is difficult although the insertion through a perforation is easy. The J-shaped stylet has a drawback in that, although selecting the blood vessel correctly at the first branch is easy, selecting the blood vessel correctly at the second branch beyond the first branch is difficult. The steerable stylet has a drawback in that the stylet tends to be expensive and the distal tip portion tends to become hard due to the complicated mechanism although the blood vessel is selected correctly by suitably manipulating the distal tip portion every time the distal tip reaches a branch and the stylet can be pushed into the blood vessel correctly. Therefore, development of a medical apparatus for insertion into a body cavity which can be manipulated easily and can deliver the distal tip to the object portion easily and safely has been attempted.

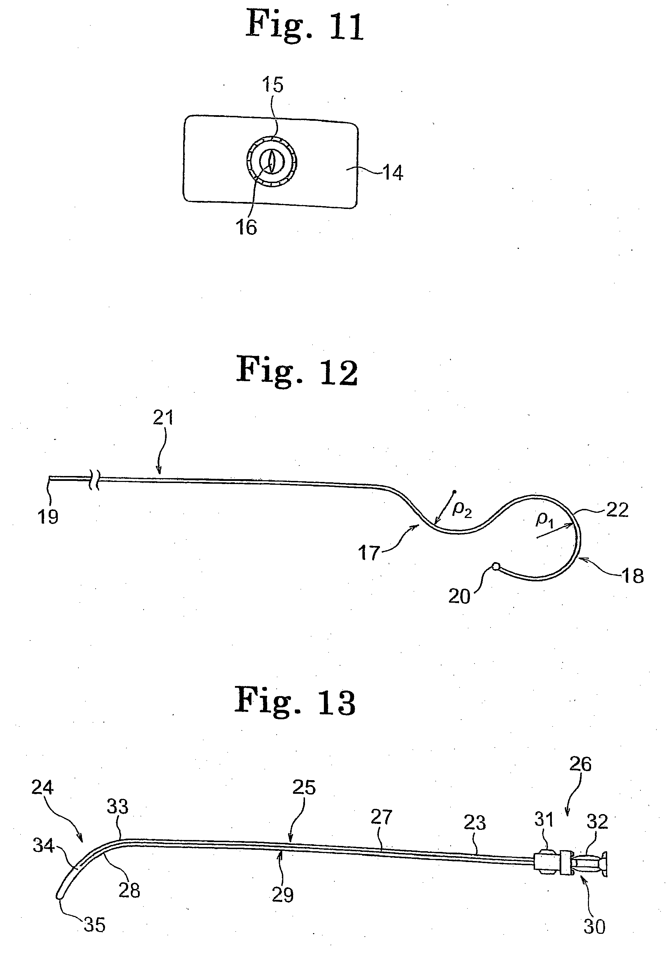

For example, as the stylet unit which provides a suitable J-shape to the free end portion of an electrode cable in the atrium so that introduction of the end portion of the cable into the auricle of the atrium and fixing of the contacting electrode to the pole of the auricle of the atrium are facilitated, a stylet unit having an inner stylet in which a stylet portion having a shape predesignated in advance is formed at a portion before the free end portion which is bent in advance and the radius of curvature of the stylet portion is in the opposite direction to the radius of curvature of the free end portion, is proposed (Patent Reference 1). Figure 12 shows a diagram exhibiting the side view of an example of the above stylet unit. The radius of curvature ρ₂ of the stylet portion 17 is in the direction opposite to the direction of the radius of curvature ρ₁ of the free end portion 18 which is bent in advance. When the shape of the stylet unit is viewed along the stylet 21 from the distal end portion 19 of the stylet to a stopping ball 20, the radius of curvature ρ₂ of the stylet portion 17 is at the left hand side of the stylet, and the radius of curvature ρ₁ of the end portion 22 is at the right hand side of the stylet.

As the catheter having a stylet which can be inserted into a blood vessel having branches and, in particular, into the ascending main vein in the high calorie infusion therapy accurately without straying into other blood vessels and without causing damages to the blood vessel, a catheter having a stylet in which a flexible catheter having a distal tip portion, a main portion and a base end portion and a stylet having a distal tip portion inserted through the inner cavity of the catheter, a main portion and a base end portion are connected to each other at the base end portion of the catheter and the base end portion of the stylet, the distal tip portion of the catheter and the distal tip portion of the stylet are curved at approximately the same position in accordance with the shape of the blood vessel at the object portion of the insertion, and the distal tip of the stylet does not protrude from the distal tip of the catheter, is proposed (Patent Reference 2). Figure 13 shows a diagram exhibiting the side view of an example of the above catheter having a stylet. The catheter 23 is composed of a distal tip portion 24, a main portion 25 and a base portion 26. The stylet 27 is composed of a distal tip portion 28, a main portion 29 and a base end portion 30. The stylet 27 is inserted through the inner cavity of the catheter 23, and a catheter connector 31 and a stylet connector 32 are fitted to each other at the base end portion. At the distal tip portion, the distal tip portion of the catheter 24 and the main portion of the catheter 25 are bent at approximately the same positions as the positions of the distal tip portion of the stylet 28 and the main portion of the stylet 29, respectively. The catheter has a second curved portion 34 at the distal side relative to the first curved portion 33. The stylet 27 does not protrude from the tip of the catheter 35.

However, even when the above stylet unit or the above catheter having a stylet is used, complicated manipulation is necessary and excellent skill is required to deliver the distal tip to the object portion of a living organ.

To achieve an easier manipulation, Patent Reference 3 (WO 99/ 23958A1) discloses a guide wire with a helical distal guide section. Similarly, Patent Reference 4 (US 2005/113862A1) provides a guide wire with steerable section. Furthermore, Patent Reference 5 (US 5295493A) provides an apparatus for intoducing an atherectomy cutter into a coronary artery for removing a stenosis from the artery, including a guide wire having a predetermined anatomically shaped configuration. Patent Reference 6 (EP 0745407A2) provides a guiding introducer for use in the treatment of atrial flutter and/or atrial fibrillation in the right atrium, having a first section of generally straight section, the second section of C-shaped section, the third section. And Patent Reference 7 (US 6558368B1) provides a catheter comprising a elongate catheter body being bendably formed near the distal end to impinge against the wall of the aorta opposite the ostium of the coronary artery.

However, further improved ease of insertion into a body cavity is also required, especially the insertion from the subclavian vein to the Bachmann's bundle, the septurm, or the pulmonary artery.
[Patent Reference 1] Japanese Patent Application Laid-Open No. Heisei 9(1997)-173464
[Patent Reference 2] Japanese Patent Application Laid-Open No. 2003-284780
[Patent Reference 3] PCT Application Laid-Open No. WO 99/ 23958A1
[Patent Reference 4] United States Patent Application Laid-Open No. 2005/113862A1
[Patent Reference 5] United States Patent Application Laid-Open No. 5295493A
[Patent Reference 6] European Patent Application Laid-Open No. 0745407A2
[Patent Reference 7] United States Patent Laid-Open No. 6558368B1

### DISCLOSURE OF THE INVENTION

The present invention as defined in claims 1-8 has an object of providing a medical apparatus for insertion into a body cavity which can spontaneously control the direction thereof and deliver the distal tip to a predesignated object portion in a living organ accurately and easily by pushing the apparatus into a body cavity.

As the result of intensive studies by the present inventors to achieve the above object, it was found that, in a medical apparatus for insertion into a body cavity which comprises a linear elastic body and can deliver the distal tip to a predesignated object portion of a living organ, the property of spontaneously controlling the direction could be provided to the medical apparatus for insertion into a body cavity and the distal tip of the medical apparatus for insertion into a body cavity proceeded along the predesignated route in the body cavity and reached the predesignated object portion of the living organ by just pushing the proximal portion of the medical apparatus for insertion into a body cavity without transfer of a torque by rotation of the proximal portion of the apparatus when curved portions were formed three-dimensionally between the proximal side and the distal side of the elastic linear body in accordance with the shape of the body cavity from the open portion or the perforation of the body cavity to the predesignated object portion. The present invention has been completed based on the knowledge.
(1) A medical apparatus for insertion into a body cavity which comprises an elastic linear body and delivers a distal tip to a predesignated object portion in a living organ, wherein the elastic linear body comprises a plurality of curved portions between a proximal side and a distal side of the elastic linear body, and at least one pair of adjacent curved portions are present on different planes which intersect each other;
(2) The medical apparatus for insertion into a body cavity described in (1), wherein, among the plurality of curved portions, a curved portion disposed closer to the distal tip has a radius of curvature smaller than a radius of curvature of a curved portion disposed closer to the proximal portion;
(3) The medical apparatus for insertion into a body cavity described in any one of (1) and (2), wherein a maximum value of the radius of curvature of a curved portion is 80 mm or greater and a minimum value of the radius of curvature of a curved portion is 40 mm or smaller;
(4) The medical apparatus for insertion into a body cavity described in any one of (1) to (3), which is a stylet in which the elastic linear body is a metal wire having a diameter of 0.1 to 1 mm, and a handle is disposed at the proximal portion;
(5) The medical apparatus for insertion into a body cavity described in any one of (1) to (3), which is a catheter in which the elastic linear body comprises an elastic macromolecular tubular body and a core wire inserted through the tubular body; and
(6) The medical apparatus for insertion into a body cavity described in any one of (1) to (5) wherein at least a portion of the elastic linear body comprises a shape memory material, and a radius of curvature of the portion comprising the shape memory material decreases when the portion is inserted into the body cavity.
(7) The medical apparatus for insertion into a body cavity described in (1), which is a stylet having two curved portions and inserted from a left subclavian vein to Bachmann's bundle as the object portion, wherein a length of the entire stylet is 442 to 662 mm, a first curved portion is curved in a leftward direction, directions of curve of the first curved portion and a second curved portion are same with each other, radius of curvature of the first curved portion is 80.5 to 120.8 mm, a radius of curvature of the second curved portion is 12.6 to 18.8 mm, an angle of intersection between a plane on which the first curved portion is present and a plane on which the second curved portion is present is 250°±20°, and a distance between the plane on which the first curved portion is present and the distal tip is 24.4 to 36.6 mm;
(8) The medical apparatus for insertion into a body cavity described in (1), which is a stylet having three curved portions and inserted from a right subclavian vein to Bachmann's bundle as the object portion, wherein a length of the entire stylet is 442 to 662 mm, a first curved portion is curved in a rightward direction, directions of curve of the first curved portion and a second curved portion are same with each other, directions of curve of the second curved portion and a third curved portion are opposite to each other, a radius of curvature of the first curved portion is 90.9 to 136.4 mm, a radius of curvature of the second curved portion is 24.7 to 37.1 mm, a radius of curvature of the third curved portion is 11.5 to 17.3 mm, an angle of intersection between a plane on which the first curved portion is present and a plane on which the second curved portion is present is 0°±20°, an angle of intersection between the plane on which the second curved portion is present and a plane on which the third curved portion is present is 230°±20°, and a distance between the plane on which the first curved portion is present and the distal tip is 18.5 to 27.8 mm;
(9) The medical apparatus for insertion into a body cavity described in (1), which is a stylet having four curved portions and inserted from a left subclavian vein to a septum as the object portion, wherein a length of the entire stylet is 496 to 744 mm, a first curved portion is curved in a leftward direction, directions of curve of the first curved portion and a second curved portion are same with each other, directions of curve of the second curved portion and a third curved portion are same with each other, directions of curve of the third curved portion and a fourth curved portion are opposite to each other, a radius of curvature of the first curved portion is 66.4 to 99.6 mm, a radius of curvature of the second curved portion is 66.7 to 100.0 mm, a radius of curvature of the third curved portion is 17.4 to 26.2 mm, a radius of curvature of the fourth curved portion is 6.6 to 9.9 mm, an angle of intersection between a plane on which the first curved portion is present and a plane on which the second curved portion is present is 0°±20°, an angle of intersection between the plane on which the second curved portion is present and a plane on which the third curved portion is present is 220°±20°, an angle of intersection between the plane on which the third curved portion is present and a plane on which the fourth curved portion is present is 330°±20°, and a distance between the plane on which the first curved portion is present and the distal tip is 36.4 to 54.6 mm;
(10) The medical apparatus for insertion into a body cavity described in (1), which is a stylet having four curved portions and inserted from a right subclavian vein to a septum as the object portion, wherein a length of the entire stylet is 496 to 744 mm, a first curved portion is curved in a rightward direction, directions of curve of the first curved portion and a second curved portion are opposite to each other, directions of curve of the second curved portion and a third curved portion are same with each other, directions of curve of the third curved portion and a fourth curved portion are opposite to each other, a radius of curvature of the first curved portion is 42.7 to 64.0 mm, a radius of curvature of the second curved portion is 46.4 to 69.6 mm, a radius of curvature of the third curved portion is 18.3 to 27.4 mm, a radius of curvature of the fourth curved portion is 6.3 to 9.4 mm, an angle of intersection between a plane on which the first curved portion is present and a plane on which the second curved portion is present is 150°±20°, an angle of intersection between the plane on which the second curved portion is present and a plane on which the third curved portion is present is 230° ± 20°, an angle of intersection between the plane on which the third curved portion is present and a plane on which the fourth curved portion is present is 320° ±20°, and a distance between the plane on which the first curved portion is present and the distal tip is 48.0 to 72.0 mm;
(11) The medical apparatus for insertion into a body cavity described in (1), which is a stylet having three curved portions and inserted from a left subclavian vein to a pulmonary artery as the object portion, wherein a length of the entire stylet is 496 to 744 mm, a first curved portion is curved in a leftward direction, directions of curve of the first curved portion and a second curved portion are same with each other, directions of curve of the second curved portion and a third curved portion are same with each other, a radius of curvature of the first curved portion is 141.1 to 211.6 mm, a radius of curvature of the second curved portion is 17.3 to 26.0 mm, a radius of curvature of the third curved portion is 4.6 to 6.9 mm, an angle of intersection between a plane on which the first curved portion is present and a plane on which the second curved portion is present is 160°±20°, an angle of intersection between the plane on which the second curved portion is present and a plane on which the third curved portion is present is 160°±20°, the third curved portion has a shape of one full circle and a half, and a length between the distal tip and a point of contact of the circle of the third curved portion is 14.8 to 22.2 mm;
(12) The medical apparatus for insertion into a body cavity described in (1), which is a stylet having four curved portions and inserted from a right subclavian vein to a pulmonary artery as the object portion, wherein a length of the entire stylet is 496 to 744 mm, a first curved portion is curved in a rightward direction, directions of curve of the first curved portion and a second curved portion are same with each other, directions of curve of the second curved portion and a third curved portion are opposite to each other, directions of curve of the third curved portion and a fourth curved portion are same with each other, a radius of curvature of the first curved portion is 114.7 to 172.0 mm, a radius of curvature of the second curved portion is 27.2 to 40.8 mm, a radius of curvature of the third curved portion is 18.1 to 27.2 mm, a radius of curvature of the fourth curved portion is 4.6 to 6.9 mm, an angle of intersection between a plane on which the first curved portion is present and a plane on which the second curved portion is present is 0° ± 20°, an angle of intersection between the plane on which the second curved portion is present and a plane on which the third curved portion is present is 140°±20°, an angle of intersection between the plane on which the third curved portion is present and a plane on which the fourth curved portion is present is 190° ±20°, the fourth curved portion has a shape of one full circle and a half, and a length between the distal tip and a point of contact of the circle is 16.0 to 24.0 mm;
(13) The medical apparatus for insertion into a body cavity described in (1), which is a stylet having two curved portions and inserted from a left subclavian vein to an auricle as the object portion, wherein a length of the entire stylet is 492 to 738 mm, a first curved portion is curved in a leftward direction, directions of curve of the first curved portion and a second curved portion are same with each other, a radius of curvature of the first curved portion is 93.9 to 140.8 mm, a radius of curvature of the second curved portion is 19.9 to 29.8 mm, an angle of intersection between a plane on which the first curved portion is present and a plane on which the second curved portion is present is 150°±20°, and a distance between the plane on which the first curved portion is present and the distal tip is 20.0 to 30.0 mm; and
(14) The medical apparatus for insertion into a body cavity described in (1), which is a stylet having three curved portions and inserted from a right subclavian vein to an auricle as the object portion, wherein a length of the entire stylet is 496 to 744 mm, a first curved portion is curved in a rightward direction, directions of curve of the first curved portion and a second curved portion are same with each other, directions of curve of the second curved portion and a third curved portion are opposite to each other, a radius of curvature of the first curved portion is 96.0 to 144.0 mm, a radius of curvature of the second curved portion is 36.8 to 55.2 mm, a radius of curvature of the third curved portion is 16.8 to 25.2 mm, an angle of intersection between a plane on which the first curved portion is present and a plane on which the second curved portion is present is 0°± 20°, an angle of intersection between the plane on which the second curved portion is present and a plane on which the third curved portion is present is 140°±20°, and a distance between the plane on which the first curved portion is present and the distal tip is 28.9 to 43.4 mm.

### BRIEF DESCRIPTION OF THE DRAWINGS

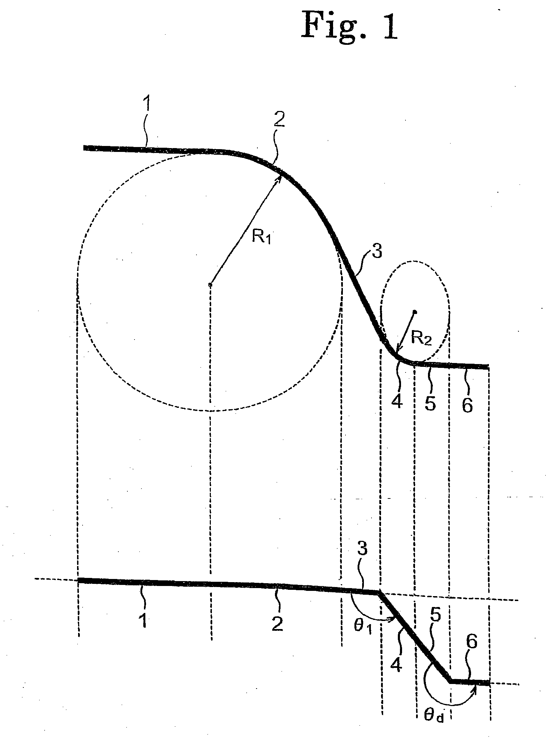

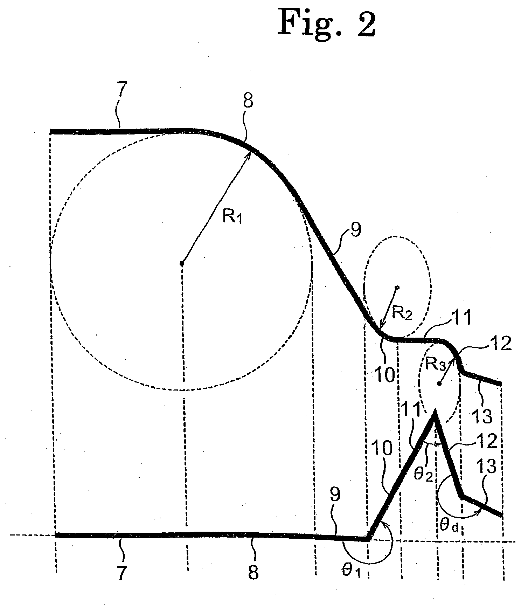

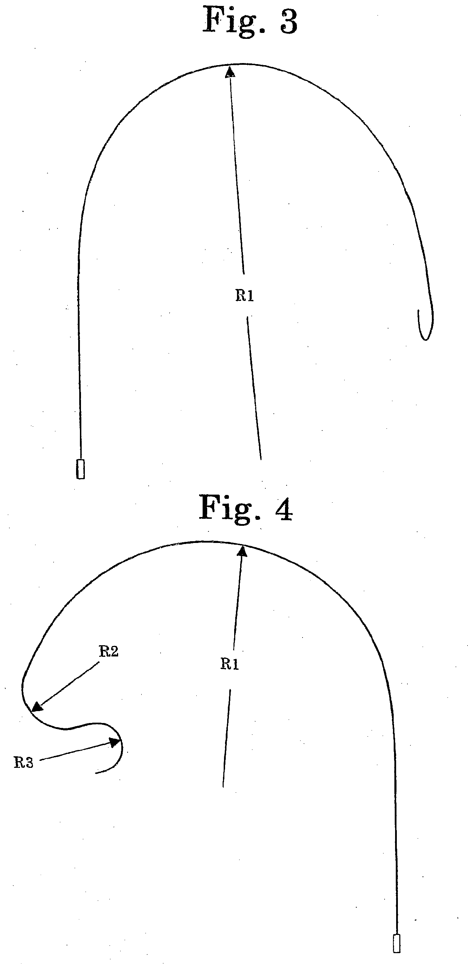

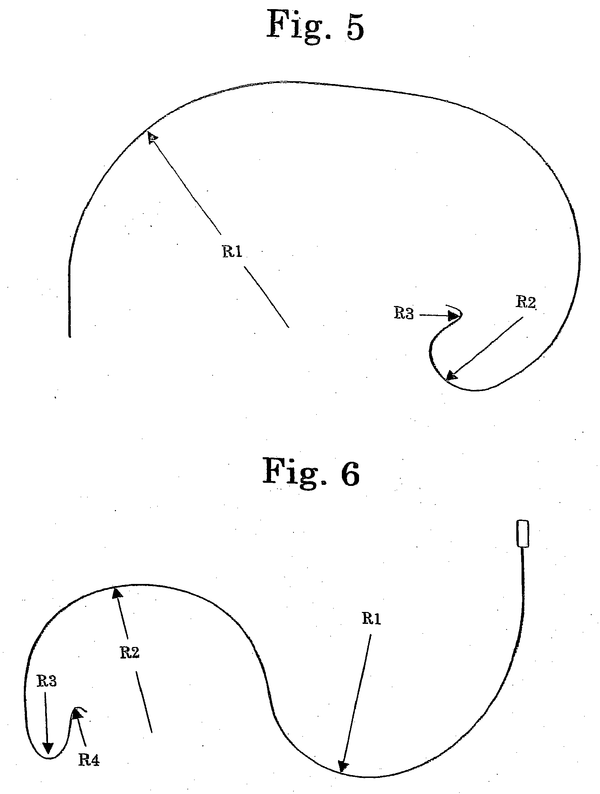

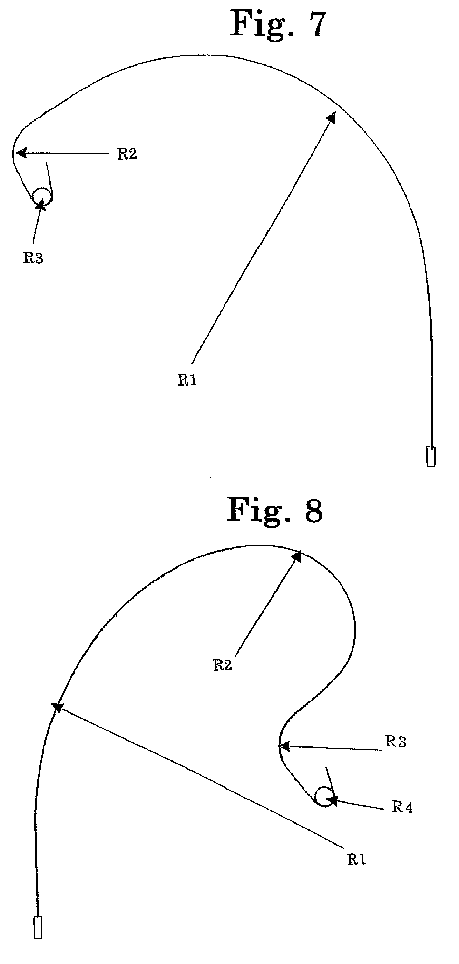

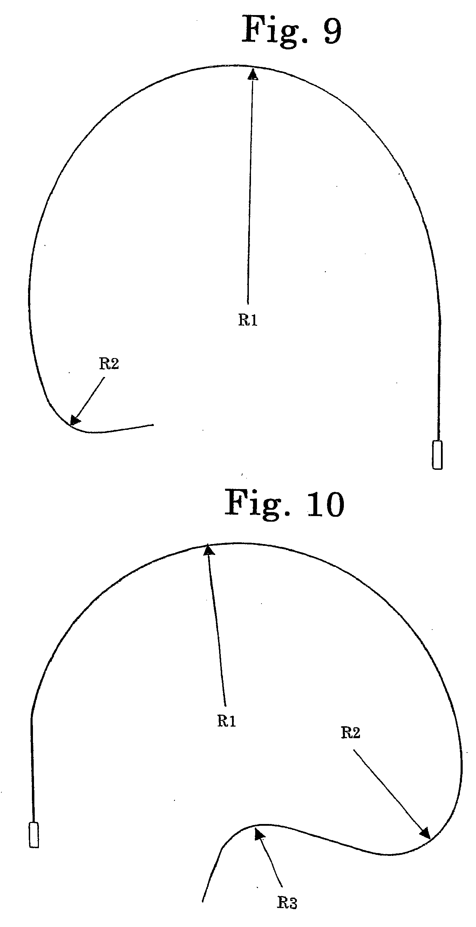

Figure 1 shows diagrams exhibiting a side view and a plan view of an embodiment of the medical apparatus for insertion into a body cavity of the present invention. Figure 2 shows diagrams exhibiting a side view and a plan view of another embodiment of the medical apparatus for insertion into a body cavity of the present invention. Figure 3 shows a diagram exhibiting a side view of an embodiment of the stylet of the present invention. Figure 4 shows a diagram exhibiting a side view of another embodiment of the stylet of the present invention. Figure 5 shows a diagram exhibiting a side view of still another embodiment of the stylet of the present invention. Figure 6 shows a diagram exhibiting a side view of still another embodiment of the stylet of the present invention. Figure 7 shows a diagram exhibiting a side view of still another embodiment of the stylet of the present invention. Figure 8 shows a diagram exhibiting a side view of still another embodiment of the stylet of the present invention. Figure 9 shows a diagram exhibiting a side view of still another embodiment of the stylet of the present invention. Figure 10 shows a diagram exhibiting a side view of still another embodiment of the stylet of the present invention. Figure 11 shows a diagram exhibiting a jig for pushing the stylet used in Examples into a body cavity. Figure 12 shows a diagram exhibiting a side view of an example of a conventional stylet unit. Figure 13 shows a diagram exhibiting a side view of an example of a conventional catheter having a stylet.

In the Figures, reference numerals mean as follows: 1: a linear portion at a proximal portion; 2: a first curved portion; 3: a first intermediate linear portion; 4: a second curved portion; 5: a second intermediate linear portion; 6: a linear portion at a distal tip; 7: a linear portion at a proximal portion; 8: a first curved portion; 9: a first intermediate linear portion; 10: a second curved portion; 11: a second intermediate linear portion; 12: a third curved portion; 13: a linear portion at a distal tip; 14: a pushing plate; 15: a ball bearing; 16: a clip; 17: a stylet portion; 18: a free end portion; 19. a base end; 20: a stopping ball; 21: a stylet; 22: a distal tip portion; 23: a catheter; 24: a distal tip portion; 25: a main portion; 26: a base end portion; 27: a stylet; 28: a distal tip portion; 29: a main portion; 30: a base end portion; 31: a catheter connector; 32: a stylet connector; 33: a first curved portion; 34: a second curved portion; and 35: a distal tip.

### THE MOST PREFERRED EMBODIMENT TO CARRY OUT THE INVENTION

The medical apparatus for insertion into a body cavity of the present invention comprises an elastic linear body and delivers the distal tip to a predesignated object portion in a living organ, wherein the elastic linear body comprises a plurality of curved portions between the proximal side and the distal side, and at least one pair of adjacent curved portions are present on different planes which intersect each other.

Figure 1 shows diagrams exhibiting a side view and a plan view of an embodiment of a medical apparatus for insertion into a body cavity of the present invention which is not in accordance with the subject matters of the claims. The medical apparatus for insertion into a body cavity of the present embodiment comprises a linear portion at the proximal portion 1, a first curved portion 2 having a radius of curvature R₁, a first intermediate linear portion 3, a second curved portion 4 having a radius of curvature R₂, a second intermediate linear portion 5 and a linear portion at the distal tip 6 between the proximal side and the distal side the linear elastic body. A plane on which the first curved portion is present and a plane on which the second curved portion is present intersect each other at an angle of θ₁, and the plane on which the second curved portion is present and the linear portion at the distal tip intersect each other at an angle of θ_{d}. The linear portion at the proximal portion and the first intermediate linear portion are present on the plane on which the first curved portion is present. The second intermediate linear portion is present on the plane on which the second curved portion is present.

Figure 2 shows diagrams exhibiting a side view and a plan view of another embodiment of a medical apparatus for insertion into a body cavity of the present invention which is not in accordance with the subject matters of the claims. The medical apparatus for insertion into a body cavity of the present embodiment comprises a linear portion at the proximal portion 7, a first curved portion 8 having a radius of curvature R₁, a first intermediate linear portion 9, a second curved portion 10 having a radius of curvature R₂, a second intermediate linear portion 11, a third curved portion 12 having a radius of curvature R₃ and a linear portion at the distal tip 13 between the proximal side and the distal side of the linear elastic body. A plane on which the first curved portion is present and a plane on which the second curved portion is present intersect each other at an angle of θ₁, the plane on which the second curved portion is present and a plane on which the third curved portion is present intersect each other at an angle of θ₂, and the plane on which the third curved portion is present and the linear portion at the distal tip intersect each other at an angle of θ_{d}. The linear portion at the proximal portion and the first intermediate linear portion are present on the plane on which the first curved portion is present. The second intermediate linear portion is present on the plane on which the second curved portion is present.

In the present invention, it is not necessary that the shape of a curved portion is a perfect arc shape but may be an approximate arc shape in accordance with the shape of the body cavity. The radius of curvature of an approximate arc shape can be obtained as the average value of the minimum radius of curvature and the maximum radius of curvature of the approximate arc shape.

In the present invention, the angle of intersection of planes on which a pair of adjacent curved portions are present can be obtained as follows. A plane on which the n-th curved portion is present and a plane on which the (n+1)-th plane is present are disposed in a manner such that the plane on which the n-th curved portion is present is placed at the proximal side and the plane on which the (n+1)-th plane is present is placed at the distal side. The angle formed with the plane on which the n-th curved portion is present and the plane on which the (n+1)-th plane is present with respect to the line of intersection of the two planes as measured in the counter-clockwise direction is defined as the angle of intersection of the planes.

Examples of the elastic linear body used in the present invention include stainless steel wires, platinum wires, gold wires and nickel-titanium alloy wires. Examples of the living organ to which the medical apparatus for insertion into a body cavity of the present invention is applied include the heart, the large vein, the trachea, the lung and the bladder. The medical apparatus for insertion into a body cavity of the present invention can be advantageously used as a preshaped stylet facilitating selective insertion of a catheter, a combination of a catheter with a preshaped stylet, a sheathing, a guide wire or a cannula. In particular, the medical apparatus for insertion into a body cavity of the present invention can be advantageously used as a stylet for an indwelling electrode for a cardiac pacemaker which works to replace the function of the living organ or a catheter for examination and curing of heart diseases.

In the medical apparatus for insertion into a body cavity of the present invention, it is preferable that, among the plurality of curved portions, a curved portion disposed closer to the distal side has a radius of curvature smaller than a radius of curvature of a curved portion disposed closer to the proximal side. When the medial apparatus is inserted into a body cavity from the outside and the distal tip is delivered to the predesignated object portion of a living organ, in general, the radius of curvature of the body cavity decreases as the distal tip approaches the object portion. For example, when a stylet is inserted by cutting open or perforating the subclavian vein to introduce an electrode for a cardiac pacemaker via the ascending large vein, and the tip of the electrode is lead to the right atrium or the right ventricle and disposed at the object portion such as the Bachmann's bundle, the septum, the pulmonary artery or the auricle, the radius of curvature of the blood vessel decreases as the tip of the electrode approaches the object portion away from the subclavian vein. When the medical apparatus for insertion into a body cavity is shaped in a manner such that a curved portion disposed closer to the distal side has a radius of curvature smaller than the radius of curvature of a curved portion disposed closer to the proximal side, the medical apparatus for insertion into a body cavity exhibits the property of spontaneously controlling the direction in accordance with the shape of the body cavity. The medical apparatus for insertion into a body cavity selects the direction correctly and proceeds by simply pushing the medical apparatus into the body cavity through the cut open portion or the perforation without adding any torque of rotation at the proximal portion of the medical apparatus, and the distal tip is delivered accurately to the predesignated object portion of the living organ without straying into other branches of the body cavity. For example, when a first curved portion having a radius of curvature close to the first curved shape in the body cavity is formed and a second curved portion is formed on a plane on which the branch of the next body cavity is present and which is disposed at a specific angle of intersection with respect to the plane on which the first curved portion is present, the second curved portion of the medical apparatus for insertion into a body cavity is already in the direction of the next branch in the body cavity as the medical apparatus for insertion into a body cavity is automatically placed along the first curved portion of the body cavity. Therefore, the distal tip of the medical apparatus for insertion into a body cavity can be placed in the correct direction in the body cavity due to the internal rotating force without applying any torque of rotation at the portion of insertion, and the direction can be easily controlled.

In the medical apparatus for insertion into a body cavity of the present invention, it is preferable that the maximum value of the radius of curvature of a curved portion is 80 mm or greater and more preferably 90 mm or greater, and the minimum value of the radius of curvature of a curved portion is 40 mm or smaller and preferably 20 mm or smaller. When the medical apparatus is inserted from the outside, in many cases, the radius of curvature of the first curved portion in the body cavity is 80 mm or greater, and the radius of curvature of the curved portion closest to the object portion is 40 mm or smaller. Therefore, the distal tip of the medical apparatus for insertion into a body cavity can be smoothly and easily delivered to the predesignated object portion of the living organ by adjusting the maximum value of the radius of curvature of a curved portion of the medical apparatus for insertion into a body cavity to 80 mm or greater and the minimum value of the radius of curvature of a curved portion to 40 mm or smaller.

The medical apparatus for insertion into a body cavity of the present invention may be a stylet in which the elastic linear body is a metal wire having a diameter of 0.1 to 1 mm and preferably 0.2 to 0.6 mm and a handle is disposed at the proximal portion. When the diameter of the elastic linear body is smaller than 0.1 mm, there is the possibility that rigidity necessary for pushing the stylet into the body cavity is insufficient. When the diameter of the elastic linear body exceeds 1 mm, rigidity is excessively great, and there is the possibility that proceeding through curved portions of the body cavity with deformation becomes difficult. The shape of the handle at the proximal portion is not particularly limited. It is preferable that the handle has a columnar shape. It is preferable that the length of the handle having a columnar shape is 10 to 60 mm and more preferably 20 to 50 mm. It is preferable that the outer diameter of the handle having a columnar shape is 4 to 20 mm and more preferably 6 to 12 mm. When the medical apparatus for insertion into a body cavity of the present invention is a stylet, the stylet exhibits the property of spontaneously controlling the direction and proceeds through the body cavity with rotation along curves of the body cavity. It is sufficient that the handle at the proximal portion is pushed forward in accordance with the spontaneous rotation of the stylet and, therefore, the handle having a columnar shape is advantageously used. The material for the handle at the proximal portion is not particularly limited. Examples of the material include polyamides and polycarbonates.

The medical apparatus for insertion into a body cavity of the present invention may be a catheter in which the elastic linear body comprises an elastic macromolecular tubular body and a core wire inserted through the tubular body. The material of the elastic macromolecular tubular body is not particularly limited. Examples of the material of the elastic macromolecular tubular body include polyethylene, polyamides and polyurethanes. The material of the core wire is not particularly limited. Examples of the core wire include stainless steel wires and nickel-titanium alloy wires. The proceeding into the body cavity can be facilitated when rigidity is decreased by decreasing the diameter of the core wire in portions closer to the distal tip portion, for example, by forming a tapered shape having the diameter decreasing from 0.35 mm to 0.02 mm. A sphere is attached by welding to the distal tip of the core wire so that damages on the inner lumen is prevented during insertion of the core wire into the elastic macromolecular tubular body.

The medical apparatus for insertion into a body cavity of the present invention may have a construction such that at least a portion of the elastic linear body comprises a shape memory material, and the radius of curvature of the portion comprising the shape memory material decreases when the portion is inserted into the body cavity. In this case, the radius of curvature of the elastic linear body described above is the radius of curvature decreased after being inserted into the body cavity. The insertion into the body cavity can be facilitated due to the greater radius of curvature, making the shape closer to the straight line, before the apparatus is inserted into the body cavity, and the property of spontaneously controlling the direction is exhibited by forming the curved portion having the predesignated radius of curvature after being inserted into the body cavity. Examples of the shape memory material used above include nickel-titanium-based shape memory alloys and iron-manganese-silicon-based shape memory alloys.

In a first embodiment, the medical apparatus for insertion into a body cavity of the present invention is a stylet having two curved portions and inserted from the left subclavian vein to the Bachmann's bundle as the object portion. The length of the entire stylet is 442 to 662 mm, more preferably 497 to 607 mm and most preferably 524 to 580 mm. The first curved portion is curved in the leftward direction, and the directions of curve of the first curved portion and the second curved portion are the same with each other. The radius of curvature of the first curved portion is 80.5 to 120.8 mm, more preferably 90.6 to 110.7 mm and most preferably 95.6 to 105.7 mm. The radius of curvature of the second curved portion is 12.6 to 18.8 mm, more preferably 14.1 to 17.3 mm and most preferably 14.9 to 16.5 mm. The angle of intersection between the plane on which the first curved portion is present and the plane on which the second curved portion is present is 250°± 20°, more preferably 250°±10° and most preferably 250°±5°. The length of the linear portion at the distal tip is 5 to 15 mm. The angle of intersection between the plane on which the second curved portion is present and the linear portion at the distal tip is -20° to +20° and more preferably -10° to +10°. The distance between the plane on which the first curved portion is present and the distal tip is 24.4 to 36.6 mm, more preferably 27.5 to 33.6 mm and most preferably 29.0 to 32.0 mm. Figure 3 shows a diagram exhibiting the side view of the stylet of the present embodiment.

In a second embodiment the medical apparatus for insertion into a body cavity of the present invention is a stylet having three curved portions and inserted from the right subclavian vein to the Bachmann's bundle as the object portion. The length of the entire stylet is 442 to 662 mm, more preferably 497 to 607 mm and most preferably 524 to 580 mm. The first curved portion is curved in the rightward direction. The directions of curve of the first curved portion and the second curved portion are the same with each other, and the directions of curve of the second curved portion and the third curved portion are opposite to each other. The radius of curvature of the first curved portion is 90.9 to 136.4 mm, more preferably 102.3 to 125.0 mm and most preferably 108.0 to 119.3 mm. The radius of curvature of the second curved portion is 24.7 to 37.1 mm, more preferably 27.8 to 34.0 mm and most preferably 29.4 to 32.5 mm. The radius of curvature of the third curved portion is 11.5 to 17.3 mm, more preferably 13.0 to 15.9 mm and most preferably 13.7 to 15.2 mm. The angle of intersection between the plane on which the first curved portion is present and the plane on which the second curved portion is present is 0°±20°, more preferably 0°±10° and most preferably 0°±5°. The angle of intersection between the plane on which the second curved portion is present and the plane on which the third curved portion is present is 230°±20°, more preferably 230°±10° and most preferably 230°±5°. The length of the linear portion at the distal tip is 5 to 15 mm. The angle of intersection between the plane on which the third curved portion is present and the linear portion at the distal tip is -20° to +20° and more preferably -10° to +10°. The distance between the plane on which the first curved portion is present and the distal tip is 18.5 to 27.8 mm, more preferably 20.8 to 25.5 mm and most preferably 22.0 to 24.3 mm. Figure 4 shows a diagram exhibiting the side view of the stylet of the present embodiment.

In a third embodiment, the medical apparatus for insertion into a body cavity of the present invention is a stylet having four curved portions and inserted from the left subclavian vein to the septum as the object portion. The length of the entire stylet is 496 to 744 mm, more preferably 558 to 682 mm and most preferably 589 to 651 mm. The first curved portion is curved in the leftward direction. The directions of curve of the first curved portion and the second curved portion are the same with each other, the directions of curve of the second curved portion and the third curved portion are the same with each other, and the directions of curve of the third curved portion and the fourth curved portion are opposite to each other. The radius of curvature of the first curved portion is 66.4 to 99.6 mm, more preferably 74.7 to 91.3 mm and most preferably 78.9 to 87.2 mm. The radius of curvature of the second curved portion is 66.7 to 100.0 mm, more preferably 75.0 to 91.7 mm and most preferably 79.2 to 87.5 mm. The radius of curvature of the third curved portion is 17.4 to 26.2 mm, more preferably 19.6 to 24.0 mm and most preferably 20.7 to 22.9 mm. The radius of curvature of the fourth curved portion is 6.6 to 9.9 mm, more preferably 7.4 to 9.1 mm and most preferably 7.8 to 8.6 mm. The angle of intersection between the plane on which the first curved portion is present and the plane on which the second curved portion is present is 0°±20°, more preferably 0°±10° and most preferably 0°±5°. The angle of intersection between the plane on which the second curved portion is present and the plane on which the third curved portion is present is 220°±20°, more preferably 220°±10 and most preferably 220° ±5. The angle of intersection between the plane on which the third curved portion is present and the plane on which the fourth curved portion is present is 330° ± 20°, more preferably 330° ± 10° and most preferably 330°±5°. The length of the linear portion at the distal tip is 10 to 20 mm. The angle of intersection between the plane on which the fourth curved portion is present and the linear portion at the distal tip is -20° to +20° and more preferably -10° to +10°. The distance between the plane on which the first curved portion is present and the distal tip is 36.4 to 54.6 mm, more preferably 41.0 to 50.1 mm and most preferably 43.2 to 47.8 mm. Figure 5 shows a diagram exhibiting the side view of the stylet of the present embodiment.

In a fourth embodiemnt, the medical apparatus for insertion into a body cavity of the present invention is a stylet having four curved portions and inserted from the right subclavian vein to the septum as the object portion. The length of the entire stylet is 496 to 744 mm, more preferably 558 to 682 mm and most preferably 589 to 651 mm. The first curved portion is curved in the rightward direction. The directions of curve of the first curved portion and the second curved portion are opposite to each other, the directions of curve of the second curved portion and the third curved portion are the same with each other, and the directions of curve of the third curved portion and the fourth curved portion are opposite to each other. The radius of curvature of the first curved portion is 42.7 to 64.0 mm, more preferably 48.0 to 58.7 mm and most preferably 50.7 to 56.0 mm. The radius of curvature of the second curved portion is 46.4 to 69.6 mm, more preferably 52.2 to 63.8 mm and most preferably 55.1 to 60.9 mm. The radius of curvature of the third curved portion is 18.3 to 27.4 mm, more preferably 20.6 to 25.2 mm and most preferably 21.7 to 24.0 mm. The radius of curvature of the fourth curved portion is 6.3 to 9.4 mm, more preferably 7.0 to 8.6 mm and most preferably 7.4 to 8.2 mm. The angle of intersection between the plane on which the first curved portion is present and the plane on which the second curved portion is present is 150° ±20°, more preferably 150°±10° and most preferably 150°±5°. The angle of intersection between the plane on which the second curved portion is present and the plane on which the third curved portion is present is 230°±20°, more preferably 230° ± 10° and most preferably 230° ± 5°. The angle of intersection between the plane on which the third curved portion is present and the plane on which the fourth curved portion is present is 320°± 20°, more preferably 320°±10° and most preferably 320°±5°. The length of the linear portion at the distal tip is 10 to 20 mm. The angle of intersection between the plane on which the fourth curved portion is present and the linear portion at the distal tip is -20° to +20° and more preferably -10° to +10°. The distance between the plane on which the first curved portion is present and the distal tip is 48.0 to 72.0 mm, more preferably 54.0 to 66.0 mm and most preferably 57.0 to 63.0 mm. Figure 6 shows a diagram exhibiting the side view of the stylet of the present embodiment.

In a fifth embodiment, the medical apparatus for insertion into a body cavity of the present invention is a stylet having three curved portions and inserted from the left subclavian vein to the pulmonary artery as the object portion. The length of the entire stylet is 496 to 744 mm, more preferably 558 to 682 mm and most preferably 589 to 651 mm. The first curved portion is curved in the leftward direction. The directions of curve of the first curved portion and the second curved portion are the same with each other, and the directions of curve of the second curved portion and the third curved portion are the same with each other. The radius of curvature of the first curved portion is 141.1 to 211.6 mm, more preferably 158.7 to 194.0 and most preferably 167.5 to 185 mm. The radius of curvature of the second curved portion is 17.3 to 26.0 mm, more preferably 19.5 to 23.8 mm and most preferably 20.6 to 22.7 mm. The radius of curvature of the third curved portion is 4.6 to 6.9 mm, more preferably 5.2 to 6.3 mm and most preferably 5.4 to 6.0 mm. The angle of intersection between the plane on which the first curved portion is present and the plane on which the second curved portion is present is 160°±20°, more preferably 160° ± 10° and most preferably 160° ± 5°. The angle of intersection between the plane on which the second curved portion is present and the plane on which the third curved portion is present is 160° ±20°, more preferably 160°±10° and most preferably 160°±5°. The length of the linear portion at the distal tip is 2 to 42 mm and more preferably 12 to 32 mm. It is preferable that the third curved portion has a shape of one full circle and a half, and the length between the distal tip and the point of contact of the circle is 14.8 to 20.4 mm, more preferably 16.7 to 20.4 mm and most preferably 17.6 to 19.4 mm. Figure 7 shows a diagram exhibiting the side view of the stylet of the present embodiment.

In a sixth embodiment, the medical apparatus for insertion into a body cavity of the present invention is a stylet having four curved portions and inserted from the right subclavian vein to the pulmonary artery as the object portion. The length of the entire stylet is 496 to 744 mm, more preferably 558 to 682 mm and most preferably 589 to 651 mm. The first curved portion is curved in the rightward direction. The directions of curve of the first curved portion and the second curved portion are the same with each other, the directions of curve of the second curved portion and the third curved portion are opposite to each other, and the directions of curve of the third curved portion and the fourth curved portion are the same with each other. The radius of curvature of the first curved portion is 114.7 to 172.0 mm, more preferably 129.0 to 157.7 mm and most preferably 136.2 to 150.5 mm. The radius of curvature of the second curved portion is 27.2 to 40.8 mm, more preferably 30.6 to 37.4 mm and most preferably 32.3 to 35.7 mm. The radius of curvature of the third curved portion is 18.1 to 27.2 mm, more preferably 20.4 to 24.9 mm and most preferably 21.5 to 23.8 mm. The radius of curvature of the fourth curved portion is 4.6 to 6.9 mm, more preferably 5.2 to 6.3 mm and more preferably 5.4 to 6.0 mm. The angle of intersection between the plane on which the first curved portion is present and the plane on which the second curved portion is present is 0°±20°, more preferably 0°±10° and most preferably 0°±5°. The angle of intersection between the plane on which the second curved portion is present and the plane on which the third curved portion is present is 140°±20°, more preferably 140°± 10° and most preferably 140°±5°. The angle of intersection between the plane on which the third curved portion is present and the plane on which the fourth curved portion is present is 190° ± 20°, more preferably 190°±10° and most preferably 190°±5°. It is preferable that the fourth curved portion has a shape of one full circle and a half, and the length between the distal tip and the point of contact of the circle is 16.0 to 24.0 mm, more preferably 18.0 to 22.0 mm and most preferably 19.0 to 21.0 mm. Figure 8 shows a diagram exhibiting the side view of the stylet of the present embodiment.

In a seventh embodiment, the medical apparatus for insertion into a body cavity of the present invention is a stylet having two curved portions and inserted from the left subclavian vein to the auricle as the object portion. The length of the entire stylet is 492 to 738 mm, more preferably 554 to 677 mm and most preferably 584 to 646 mm. The first curved portion is curved in the leftward direction. The directions of curve of the first curved portion and the second curved portion are the same with each other. The radius of curvature of the first curved portion is 93.9 to 140.8 mm, more preferably 105.5 to 129.1 mm and most preferably 111.5 to 123.2 mm. The radius of curvature of the second curved portion is 19.9 to 29.8 mm, more preferably 22.3 to 27.3 mm and most preferably 23.6 to 26.1 mm. The angle of intersection between the plane on which the first curved portion is present and the plane on which the second curved portion is present is 150°±20°, more preferably 150°±10° and most preferably 150°±5°. The length of the linear portion at the distal tip is 5 to 55 mm and more preferably 15 to 45 mm. The angle of intersection between the plane on which the second curved portion is present and the linear portion at the distal tip is -20° to +20° and more preferably -10° to +10°. The distance between the plane on which the first curved portion is present and the distal tip is 20.0 to 30.0 mm, more preferably 22.5 to 27.5 mm and most preferably 23.8 to 26.3 mm. Figure 9 shows a diagram exhibiting the side view of the stylet of the present embodiment.

In an eighth embodiment, the medical apparatus for insertion into a body cavity of the present invention is a stylet having three curved portions and inserted from the right subclavian vein to the auricle as the object portion. The length of the entire stylet is 496 to 744 mm, more preferably 558 to 682 mm and most preferably 589 to 651 mm. The first curved portion is curved in the rightward direction. The directions of curve of the first curved portion and the second curved portion are the same with each other, and the directions of curve of the second curved portion and the third curved portion are opposite to each other. The radius of curvature of the first curved portion is 96.0 to 144.0 mm, more preferably 108.0 to 132.0 mm and most preferably 114.0 to 126 mm. The radius of curvature of the second curved portion is 36.8 to 55.2 mm, more preferably 41,4 to 50.6 mm and most preferably 43.7 to 48.3 mm. The radius of curvature of the third curved portion is 16.8 to 25.2 mm, more preferably 18.9 to 23.1 mm and most preferably 20.0 to 22.1 mm. The angle of intersection between the plane on which the first curved portion is present and the plane on which the second curved portion is present is 0°±20°, more preferably 0° ±10° and most preferably 0°±5°. The length of the linear portion at the distal tip is 5 to 55 mm and more preferably 15 to 45 mm. The angle of intersection between the plane on which the second curved portion is present and the linear portion at the distal tip is -20° to +20° and more preferably -10° to +10°. The distance between the plane on which the first curved portion is present and the distal tip is 28.9 to 43.4 mm, more preferably 32.5 to 39.8 mm and most preferably 34.4 to 38.0 mm. Figure 10 shows a diagram exhibiting the side view of the stylet of the present embodiment.

### EXAMPLES

The present invention will be described more specifically with reference to Examples in the following. However, the present invention is not limited to the examples.

### Example 1

A stylet having a linear proximal portion, a first curved portion, a first intermediate linear portion, a second curved portion, a second intermediate linear portion and a linear portion at the distal tip was prepared by working a stainless steel (SUS 304) wire having a diameter of 0.35 mm and a length of 552 mm. The linear proximal portion, the first curved portion and the first intermediate linear portion were placed on the same plane. The length of the linear portion was adjusted to 123 mm. The first curved portion was placed in the leftward direction. The radius of curvature of the first curved portion was adjusted to 101 mm, and the angle around the center was adjusted to 163°. The length of the first intermediate linear portion was adjusted to 58 mm. On a plane intersecting the plane on which the first curved portion is present at an angle of 100°, the second curved portion having a radius of curvature of 15.7 mm and an angle around the center of 180° was placed in the same direction of curve as that of the first curved portion. In the direction at an angle of 230° with respect to the plane on which the second curved portion is present, the linear portion at the distal tip having a length of 34.6 mm was formed. In the linear portion at the distal tip, a taper was formed in a manner such that the diameter gradually decreased from 0.35 mm at the point connected to the second curved portion to 0.02 mm at the distal tip, and a sphere having a diameter of 0.28 mm was attached to the distal tip by welding. In the actual working, the curves were formed successively from the linear portion at the distal tip to the linear proximal portion. The distal tip of the stylet was placed at a position separated from the plane on which the first curved portion was present by 3.05 mm.

A model of blood vessel from the left subclavian vein to the ascending large vein was prepared by working a polyurethane tube. Branches in the blood vessel were formed in the model of the blood vessel exactly in the same shapes as those of the actual blood vessel. The ascending large vein in a transparent model of the heart [manufactured by Kyoto Kagaku Co., Ltd] was connected to the ascending large vein in the above model of the blood vessel.

A jig for pushing a stylet into a body cavity shown in Figure 11 was prepared. In the jig for pushing a stylet into a body cavity, a clip 16 having a diameter of 10 mm and freely rotating around a ball bearing 15 was disposed at the center of a pushing plate 14 made of stainless steel and having a length of 20 mm, a width of 40 mm and a thickness of 3 mm.

The linear proximal portion of the above stylet was held by the clip in the jig for pushing a stylet into a body cavity. The distal tip of the stylet was pushed into the left subclavian vein of the model of the blood vessel by the left hand and, while the left hand was kept along the stylet, the jig for pushing a stylet into a body cavity was slowly pushed forward by the right hand. Although no torque of rotation was applied to the stylet at the proximal portion, the stylet pushed into the blood vessel exhibited the property of spontaneously controlling the direction and freely rotated, and the distal tip of the stylet accurately reached the Bachmann's bundle in the transparent model of the heart without straying into other branches of the blood vessel.

### Example 2

A core wire having a linear proximal portion, a first curved portion and a second curved portion was prepared by working a wire of an ultra-elastic nickel titanium alloy having a diameter of 0.30 mm and a length of 615 mm. The linear proximal portion and the first curved portion were placed on the same plane. The length of the linear proximal portion was adjusted to 120 mm. The first curved portion was placed in the leftward direction. The radius of curvature of the first curved portion was adjusted to 117 mm, and the angle around the center was adjusted to 215°. On a plane intersecting the plane on which the first curved portion is present at an angle of 160°, the second curved portion having a radius of curvature of 24.8 mm and an angle around the center of 130° was placed in the same direction of the curve as that of the first curved portion. In the portion of the second curved portion at the distal side, a taper was formed in a manner such that the diameter gradually decreased to 0.02 mm at the distal tip, and a sphere having a diameter of 0.28 mm was attached to the distal tip by welding. The tip of the core wire was placed at a position separated from the plane on which the first curved portion was present by 25.0 mm.

The obtained core wire was inserted through a narrow tube of polyethylene of nominal number of 3Fr having an inner diameter of 0.5 mm, an outer diameter of 1 mm and a length of 615 mm, and a catheter was prepared.

### INDUSTRIAL APPLICABILITY

When the medical apparatus for insertion into a body cavity of the present invention is inserted into the body cavity, complicated operations for selecting a branch and directing the distal tip of the apparatus to the object portion by transfer of torque at the proximal portion are not necessary, and the distal tip of the apparatus can be delivered to the predesignated object portion in a living organ by simply pushing the apparatus forward at the proximal portion since the medical apparatus for insertion into a body cavity has the property of spontaneously controlling the direction. By using the medical apparatus for insertion into a body cavity of the present invention, a catheter or the like can be quickly and easily introduced to the object portion of a living organ for the many purposes such as diagnosis, treatments, operations in a blood vessel and replacements of the function of the living organ. In particular, the medical apparatus for insertion into a body cavity of the present invention is useful as the stylet for placing an indwelling electrode of a pacemaker for the heart. A lead electrode can be placed safely and accurately at the object portion of the atrium or the ventricle pervenously without skill of a great degree.

## Claims

1. A stylet which comprises an elastic linear body, said elastic linear body comprising a plurality of curved portions between a proximal side and a distal side of the elastic linear body, wherein at least one pair of adjacent curved portions are present on different planes which intersect each other, said stylet has two curved portions comprising a first curved portion disposed at the proximal side and a second curved portion disposed at the distal side, **characterized in that** an entire length of the stylet is 442 to 662 mm, the first curved portion is curved in the leftward direction, directions of curve of the first curved portion and the second curved portion are same with each other, radius of curvature of the first curved portion (R1) is 80.5 to 120.8 mm, a radius of curvature of the second curved portion is 12.6 to 18.8 mm, an angle of intersection between a plane on which the first curved portion is present and a plane on which the second curved portion is present is 250°±20° with respect to the line of intersection of the two planes as measured in the counter-clockwise direction by placing the plane on which the first curved portion is present at proximal side and the plane on which the second curved portion is present at distal side, the length of the linear portion at the distal tip is 5 to 15 mm, the angle of intersection between the plane on which the second curved portion is present and the linear portion at the distal tip is -20° to +20° and the distance between the plane on which the first curved portion is present and the distal tip is 24.4 to 36.6 mm.

2. A stylet which comprises an elastic linear body, said elastic linear body comprising a plurality of curved portions between a proximal side and a distal side of the elastic linear body, wherein at least one pair of adjacent curved portions are present on different planes which intersect each other, said stylet has three curved portions comprising a first curved portion disposed at the proximal side, a second curved portion disposed at the distal side of the first curved portion and a third curved portion disposed at the distal side of the second curved portion, **characterized in that** an entire length of the stylet is 442 to 662 mm, the first curved portion is curved in the rightward direction, directions of curve of the first curved portion and a second curved portion are same with each other, directions of curve of the second curved portion and a third curved portion are opposite to each other, a radius of curvature of the first curved portion (R1) is 90.9 to 136.4 mm, a radius of curvature of the second curved portion (R2) is 24.7 to 37.1 mm, a radius of curvature of the third curved portion (R3) is 11.5 to 17.3 mm, an angle of intersection between a plane on which the first curved portion is present and a plane on which the second curved portion is present is 0°±20° with respect to the line of intersection of the two planes as measured in the counter-clockwise direction by placing the plane on which the first curved portion is present at proximal side and the plane on which the second curved portion is present at distal side, an angle of intersection between the plane on which the second curved portion is present and a plane on which the third curved portion is present is 230°±20° with respect to the line of intersection of the two planes as measured in the counter-clockwise direction by placing the plane on which the second curved portion is present at proximal side and the plane on which the third curved portion is present at distal side, a length of the linear portion at the distal tip is 5 to 15 mm, an angle of intersection between the plane on which the third curved portion is present and the linear portion at the distal tip is -20° to +20° and a distance between the plane on which the first curved portion is present and the distal tip is 18.5 to 27.8 mm.

3. A stylet which comprises an elastic linear body, said elastic linear body comprising a plurality of curved portions between a proximal side and a distal side of the elastic linear body, wherein at least one pair of adjacent curved portions are present on different planes which intersect each other, said stylet has four curved portions comprising a first curved portion disposed at the proximal side, a second curved portion disposed at the distal side of the first curved portion, a third curved portion disposed at the distal side of the second curved portion, and a fourth curved portion disposed at the distal side of the third curved portion, **characterized in that** an entire length of the stylet is 496 to 744 mm, the first curved portion is curved in the leftward direction, directions of curve of the first curved portion and the second curved portion are same with each other, directions of curve of the second curved portion and the third curved portion are same with each other, directions of curve of the third curved portion and the fourth curved portion are opposite to each other, a radius of curvature of the first curved portion (R1) is 66.4 to 99.6 mm, a radius of curvature of the second curved portion is 66.7 to 100.0 mm, a radius of curvature of the third curved portion (R2) is 17.4 to 26.2 mm, a radius of curvature of the fourth curved portion (R3) is 6.6 to 9.9 mm, an angle of intersection between a plane on which the first curved portion is present and a plane on which the second curved portion is present is 0°±20° with respect to the line of intersection of the two planes as measured in the counter-clockwise direction by placing the plane on which the first curved portion is present at proximal side and the plane on which the second curved portion is present at distal side, an angle of intersection between the plane on which the second curved portion is present and a plane on which the third curved portion is present is 220°±20° with respect to the line of intersection of the two planes as measured in the counter-clockwise direction by placing the plane on which the second curved portion is present at proximal side and the plane on which the third curved portion is present at distal side, an angle of intersection between the plane on which the third curved portion is present and a plane on which the fourth curved portion is present is 330°±20° with respect to the line of intersection of the two planes as measured in the counter-clockwise direction by placing the plane on which the third curved portion is present at proximal side and the plane on which the fourth curved portion is present at distal side, a length of the linear portion at the distal tip is 10 to 20 mm, an angle of intersection between the plane on which the fourth curved portion is present and the linear portion at the distal tip is -20° to +20° and a distance between the plane on which the first curved portion is present and the distal tip 36.4 to 54.6 mm.

4. A stylet which comprises an elastic linear body, said elastic linear body comprising a plurality of curved portions between a proximal side and a distal side of the elastic linear body, wherein at least one pair of adjacent curved portions are present on different planes which intersect each other, said stylet has four curved portions comprising a first curved portion disposed at the proximal side, a second curved portion disposed at the distal side of the first curved portion, a third curved portion disposed at the distal side of the second curved portion and a fourth curved portion disposed at the distal side of the third curved portion, **characterized in that** an entire length of the stylet is 496 to 744 mm, the first curved portion is curved in the rightward direction, directions of curve of the first curved portion and the second curved portion are opposite to each other, directions of curve of the second curved portion and the third curved portion are same with each other, directions of curve of the third curved portion and the fourth curved portion are opposite to each other, a radius of curvature of the first curved portion (R1) is 42.7 to 64.0 mm, a radius of curvature of the second curved portion (R2) is 46.4 to 69.6 mm, a radius of curvature of the third curved portion (R3) is 18.3 to 27.4 mm, a radius of curvature of the fourth curved portion (R4) is 6.3 to 9.4 mm, an angle of intersection between a plane on which the first curved portion is present and a plane on which the second curved portion is present is 150°±20° with respect to the line of intersection of the two planes as measured in the counter-clockwise direction by placing the plane on which the first curved portion is present at proximal side and the plane on which the second curved portion is present at distal side, an angle of intersection between the plane on which the second curved portion is present and a plane on which the third curved portion is present is 230°±20° with respect to the line of intersection of the two planes as measured in the counter-clockwise direction by placing the plane on which the second curved portion is present at proximal side and the plane on which the third curved portion is present at distal side, an angle of intersection between the plane on which the third curved portion is present and a plane on which the fourth curved portion is present is 320°±20° with respect to the line of intersection of the two planes as measured in the counter-clockwise direction by placing the plane on which the third curved portion is present at proximal side and the plane on which the fourth curved portion is present at distal side, a length of the linear portion at the distal tip is 10 to 20 mm, an angle of intersection between the plane on which the fourth curved portion is present and the linear portion at the distal tip is -20° to +20° and a distance between the plane on which the first curved portion is present and the distal tip is 48.0 to 72.0 mm.

5. A stylet which comprises an elastic linear body, said elastic linear body comprising a plurality of curved portions between a proximal side and a distal side of the elastic linear body, wherein at least one pair of adjacent curved portions are present on different planes which intersect each other, said stylet has three curved portions comprising a first curved portion disposed at the proximal side, a second curved portion disposed at the distal side of the first curved portion and a third curved portion disposed at the distal side of the second curved portion, **characterized in that** an entire length of the stylet is 496 to 744 mm, the first curved portion is curved in the leftward direction, directions of curve of the first curved portion and the second curved portion are same with each other, directions of curve of the second curved portion and the third curved portion are same with each other, a radius of curvature of the first curved portion (R1) is 141.1 to 211.6 mm, a radius of curvature of the second curved portion (R2) is 17.3 to 26.0 mm, a radius of curvature of the third curved portion (R3) is 4.6 to 6.9 mm, an angle of intersection between a plane on which the first curved portion is present and a plane on which the second curved portion is present is 160°±20° with respect to the line of intersection of the two planes as measured in the counter-clockwise direction by placing the plane on which the first curved portion is present at proximal side and the plane on which the second curved portion is present at distal side, an angle of intersection between the plane on which the second curved portion is present and a plane on which the third curved portion is present is 160°±20° with respect to the line of intersection of the two planes as measured in the counter-clockwise direction by placing the plane on which the second curved portion is present at proximal side and the plane on which the third curved portion is present at distal side, the third curved portion has a shape of one full circle and a half, a length of the linear portion at the distal tip is 2 to 42 mm and a length between the distal tip and the point of contact of the circle is 14.8 to 20.4 mm.

6. A stylet which comprises an elastic linear body, said elastic linear body comprising a plurality of curved portions between a proximal side and a distal side of the elastic linear body, wherein at least one pair of adjacent curved portions are present on different planes which intersect each other, said stylet has four curved portions comprising a first curved portion disposed at the proximal side, a second curved portion disposed at the distal side of the first curved portion, a third curved portion disposed at the distal side of the second curved portion and a fourth curved portion disposed at the distal side of the third curved portion, **characterized in that** an entire length of the stylet is 496 to 744 mm, the first curved portion is curved in the rightward direction, directions of curve of the first curved portion and the second curved portion are same with each other, directions of curve of the second curved portion and the third curved portion are opposite to each other, directions of curve of the third curved portion and the fourth curved portion are same with each other, a radius of curvature of the first curved portion (R1) is 114.7 to 172.0 mm, a radius of curvature of the second curved portion (R2) is 27.2 to 40.8 mm, a radius of curvature of the third curved portion (R3) is 18.1 to 27.2 mm, a radius of curvature of the fourth curved portion (R4) is 4.6 to 6.9 mm, an angle of intersection between a plane on which the first curved portion is present and a plane on which the second curved portion is present is 0°±20° with respect to the line of intersection of the two planes as measured in the counter-clockwise direction by placing the plane on which the first curved portion is present at proximal side and the plane on which the second curved portion is present at distal side, an angle of intersection between the plane on which the second curved portion is present and a plane on which the third curved portion is present is 140°±20° with respect to the line of intersection of the two planes as measured in the counter-clockwise direction by placing the plane on which the second curved portion is present at proximal side and the plane on which the third curved portion is present at distal side, an angle of intersection between the plane on which the third curved portion is present and a plane on which the fourth curved portion is present is 190°±20° with respect to the line of intersection of the two planes as measured in the counter-clockwise direction by placing the plane on which the third curved portion is present at proximal side and the plane on which the fourth curved portion is present at distal side, the fourth curved portion has a shape of one full circle and a half and the length between the distal tip and the point of contact of the circle is 16.0 to 24.0 mm.

7. A stylet which comprises an elastic linear body, said elastic linear body comprising a plurality of curved portions between a proximal side and a distal side of the elastic linear body, wherein at least one pair of adjacent curved portions are present on different planes which intersect each other, said stylet has two curved portions comprising a first curved portion disposed at the proximal side and a second curved portions disposed at the distal side, **characterized in that** an entire length of the stylet is 492 to 738 mm, the first curved portion is curved in the leftward direction, directions of curve of the first curved portion and the second curved portion are same with each other, a radius of curvature of the first curved portion (R1) is 93.9 to 140.8 mm, a radius of curvature of the second curved portion (R2) is 19.9 to 29.8 mm, an angle of intersection between a plane on which the first curved portion is present and a plane on which the second curved portion is present is 150° ±20° with respect to the line of intersection of the two planes as measured in the counter-clockwise direction by placing the plane on which the first curved portion is present at proximal side and the plane on which the second curved portion is present at distal side, the length of the linear portion at the distal tip is 5 to 55 mm, an angle of intersection between the plane on which the second curved portion is present and the linear portion at the distal tip is -20° to +20° and the distance between the plane on which the first curved portion is present and the distal tip is 20.0 to 30.0 mm.

8. A stylet which comprises an elastic linear body, said elastic linear body comprising a plurality of curved portions between a proximal side and a distal side of the elastic linear body, wherein at least one pair of adjacent curved portions are present on different planes which intersect each other, said stylet has three curved portions comprising a first curved portion disposed at the proximal side, a second curved portion disposed at the distal side of the first curved portion and a third curved portion disposed at the distal side of the second curved portion, **characterized in that** an entire length of the entire stylet is 496 to 744 mm, the first curved portion is curved in the rightward direction, directions of curve of the first curved portion and the second curved portion are same with each other, directions of curve of the second curved portion and the third curved portion are opposite to each other, a radius of curvature of the first curved portion (R1) is 96.0 to 144.0 mm, a radius of curvature of the second curved portion (R2) is 36.8 to 55.2 mm, a radius of curvature of the third curved portion (R3) is 16.8 to 25.2 mm, an angle of intersection between a plane on which the first curved portion is present and a plane on which the second curved portion is present is 0°±20° with respect to the line of intersection of the two planes as measured in the counter-clockwise direction by placing the plane on which the first curved portion is present at proximal side and the plane on which the second curved portion is present at distal side, an angle of intersection between the plane on which the second curved portion is present and a plane on which the third curved portion is present is 140°±20° with respect to the line of intersection of the two planes as measured in the counter-clockwise direction by placing the plane on which the second curved portion is present at proximal side and the plane on which the third curved portion is present at distal side, the length of the linear portion at the distal tip is 5 to 55 mm, an angle of intersection between the plane on which the second curved portion is present and the linear portion at the distal tip is -20° to +20° and a distance between the plane on which the first curved portion is present and the distal tip is 28.9 to 43.4 mm.

9. A stylet according to any one of Claims 1 to 8, wherein the elastic linear body is a metal wire having a diameter of 0.1 to 1 mm, and a handle is disposed at the proximal end of the stylet.

10. A stylet according to any one of Claims 1 to 9, wherein the stylet is a stylet for placing an indwelling electrode of a pacemaker for heart.

## Patentansprüche

1. Mandrin, der einen elastischen linearen Körper aufweist, wobei der elastische lineare Körper eine Vielzahl von gebogenen Abschnitten zwischen einer proximalen Seite und einer distalen Seite des elastischen linearen Körpers aufweist, wobei zumindest ein Paar aneinander angrenzender gebogener Abschnitte in verschiedenen Ebenen liegt, die sich kreuzen, wobei der Mandrin zwei gebogene Abschnitte aufweist mit einem an der proximalen Seite angeordneten ersten gebogenen Abschnitt und einem an der distalen Seite angeordneten zweiten gebogenen Abschnitt, **dadurch gekennzeichnet, dass** die Gesamtlänge des Mandrins zwischen 442 und 662 mm beträgt, wobei der erste gebogene Abschnitt in Richtung nach links gebogen ist, wobei die Biegerichtungen des ersten gebogenen Abschnitts und des zweiten gebogenen Abschnitts die gleichen sind, wobei der Krümmungsradius des ersten gebogenen Abschnitts (R1) zwischen 80,5 und 120,8 mm und der Krümmungsradius des zweiten gebogenen Abschnitts zwischen 12,6 und 18,8 mm liegt, wobei ein Schnittwinkel zwischen einer Ebene, in welcher der erste gebogene Abschnitt liegt, und einer Ebene, in welcher der zweite gebogene Abschnitt liegt, 250° ± 20° beträgt bezüglich der Schnittlinie der beiden Ebenen, gemessen im Gegenuhrzeigersinn, wobei die Ebene, in welcher der erste gebogene Abschnitt liegt, auf der proximalen Seite angeordnet und die Ebene, in welcher der zweite gebogene Abschnitt liegt, auf der distalen Seite angeordnet ist, wobei die Länge des linearen Abschnitts an der distalen Spitze 5 bis 15 mm beträgt, wobei der Schnittwinkel zwischen der Ebene, in welcher der zweite gebogene Abschnitt liegt, und dem linearen Abschnitt an der distalen Spitze zwischen - 20° und + 20° beträgt und der Abstand zwischen der Ebene, in welcher der erste gebogene Abschnitt liegt, und der distalen Spitze zwischen 24,4 und 36,6 mm beträgt.

2. Mandrin, der einen elastischen linearen Körper aufweist, wobei der elastische lineare Körper eine Vielzahl von gebogenen Abschnitten zwischen einer proximalen Seite und einer distalen Seite des elastischen linearen Körpers aufweist, wobei zumindest ein Paar aneinander angrenzender gebogener Abschnitte in verschiedenen Ebenen liegt, die sich kreuzen, wobei der Mandrin drei gebogene Abschnitte aufweist mit einem an der proximalen Seite angeordneten ersten gebogenen Abschnitt, einem an der distalen Seite des ersten gebogenen Abschnitts angeordneten zweiten gebogenen Abschnitt und einem an der distalen Seite des zweiten gebogenen Abschnitts angeordneten dritten Abschnitt, **dadurch gekennzeichnet, dass** die Gesamtlänge des Mandrins zwischen 442 und 662 mm beträgt, wobei der erste gebogene Abschnitt in Richtung nach rechts gebogen ist, wobei die Biegerichtungen des ersten gebogenen Abschnitts und des zweiten gebogenen Abschnitts die gleichen sind, wobei die Biegerichtungen des zweiten gebogenen Abschnitts und des dritten gebogenen Abschnitts zueinander entgegengesetzt sind, wobei der Krümmungsradius des ersten gebogenen Abschnitts (R1) zwischen 90,9 und 136,4 mm, der Krümmungsradius des zweiten gebogenen Abschnitts (R2) zwischen 24,7 und 37,1 mm und der Krümmungsradius des dritten gebogenen Abschnitts (R3) zwischen 11,5 und 17,3 mm liegt, wobei ein Schnittwinkel zwischen einer Ebene, in welcher der erste gebogene Abschnitt liegt, und einer Ebene, in welcher der zweite gebogene Abschnitt liegt, 0° ± 20° beträgt bezüglich der Schnittlinie der beiden Ebenen, gemessen im Gegenuhrzeigersinn, wobei die Ebene, in weicher der erste gebogene Abschnitt liegt, auf der proximalen Seite angeordnet und die Ebene, in welcher der zweite gebogene Abschnitt liegt, auf der distalen Seite angeordnet ist, wobei ein Schnittwinkel zwischen der Ebene, in weicher der zweite gebogene Abschnitt liegt, und einer Ebene, in welcher der dritte gebogene Abschnitt liegt, 230° ± 20° beträgt bezüglich der Schnittlinie der beiden Ebenen, gemessen im Gegenuhrzeigersinn, wobei die Ebene, in welcher der zweite gebogene Abschnitt liegt, auf der proximalen Seite angeordnet und die Ebene, in welcher der dritte gebogene Abschnitt liegt, auf der distalen Seite angeordnet ist, wobei die Länge des linearen Abschnitts an der distalen Spitze 5 bis 15 mm beträgt, wobei der Schnittwinkel zwischen der Ebene, in welcher der dritte gebogene Abschnitt liegt, und dem linearen Abschnitt an der distalen Spitze zwischen - 20° und + 20° beträgt und der Abstand zwischen der Ebene, in welcher der erste gebogene Abschnitt liegt, und der distalen Spitze zwischen 18,5 und 27,8 mm beträgt.

3. Mandrin, der einen elastischen linearen Körper aufweist, wobei der elastische lineare Körper eine Vielzahl von gebogenen Abschnitten zwischen einer proximalen Seite und einer distalen Seite des elastischen linearen Körpers aufweist, wobei zumindest ein Paar aneinander angrenzender gebogener Abschnitte in verschiedenen Ebenen liegt, die sich kreuzen, wobei der Mandrin vier gebogene Abschnitte aufweist mit einem an der proximalen Seite angeordneten ersten gebogenen Abschnitt, einem an der distalen Seite des ersten gebogenen Abschnitts angeordneten zweiten gebogenen Abschnitt, einem an der distalen Seite des zweiten gebogenen Abschnitts angeordneten dritten gebogenen Abschnitt und einem an der distalen Seite des dritten gebogenen Abschnitts angeordneten vierten gebogenen Abschnitt, **dadurch gekennzeichnet, dass** die Gesamtlänge des Mandrins zwischen 496 und 744 mm beträgt, wobei der erste gebogene Abschnitt in Richtung nach links gebogen ist, wobei die Biegerichtungen des ersten gebogenen Abschnitts und des zweiten gebogenen Abschnitts die gleichen sind, wobei die Biegerichtungen des zweiten gebogenen Abschnitts und des dritten gebogenen Abschnitts die gleichen sind, wobei die Biegerichtungen des dritten gebogenen Abschnitts und des vierten gebogenen Abschnitts zueinander entgegengesetzt sind, wobei der Krümmungsradius des ersten gebogenen Abschnitts (R1) zwischen 66,4 und 99,6 mm, der Krümmungsradius des zweiten gebogenen Abschnitts zwischen 66,7 und 100,0 mm, der Krümmungsradius des dritten gebogenen Abschnitts (R2) zwischen 17,4 und 26,2 mm und der Krümmungsradius des vierten gebogenen Abschnitts (R3) zwischen 6,6 und 9,9 mm liegt, wobei ein Schnittwinkel zwischen einer Ebene, in welcher der erste gebogene Abschnitt liegt, und einer Ebene, in welcher der zweite gebogene Abschnitt liegt, 0° ± 20° beträgt bezüglich der Schnittlinie der beiden Ebenen, gemessen im Gegenuhrzeigersinn, wobei die Ebene, in welcher der erste gebogene Abschnitt liegt, auf der proximalen Seite angeordnet und die Ebene, in welcher der zweite gebogene Abschnitt liegt, auf der distalen Seite angeordnet ist, wobei ein Schnittwinkel zwischen der Ebene, in welcher der zweite gebogene Abschnitt liegt, und einer Ebene, in welcher der dritte gebogene Abschnitt liegt, 220° ± 20° beträgt bezüglich der Schnittlinie der beiden Ebenen, gemessen im Gegenuhrzeigersinn, wobei die Ebene, in welcher der zweite gebogene Abschnitt liegt, auf der proximalen Seite angeordnet und die Ebene, in welcher der dritte gebogene Abschnitt liegt, auf der distalen Seite angeordnet ist, wobei ein Schnittwinkel zwischen der Ebene, in welcher der dritte gebogene Abschnitt liegt, und einer Ebene, in welcher der vierte gebogene Abschnitt liegt, 330° ± 20° beträgt bezüglich der Schnittlinie der beiden Ebenen, gemessen im Gegenuhrzeigersinn, wobei die Ebene, in welcher der dritte gebogene Abschnitt liegt, auf der proximalen Seite angeordnet und die Ebene, in welcher der vierte gebogene Abschnitt liegt, auf der distalen Seite angeordnet ist, wobei die Länge des linearen Abschnitts an der distalen Spitze 10 bis 20 mm beträgt, wobei der Schnittwinkel zwischen der Ebene, in welcher der vierte gebogene Abschnitt liegt, und dem linearen Abschnitt an der distalen Spitze zwischen - 20° und + 20° beträgt und der Abstand zwischen der Ebene, in welcher der erste gebogene Abschnitt liegt, und der distalen Spitze zwischen 36,4 und 54,6 mm beträgt.

4. Mandrin, der einen elastischen linearen Körper aufweist, wobei der elastische lineare Körper eine Vielzahl von gebogenen Abschnitten zwischen einer proximalen Seite und einer distalen Seite des elastischen linearen Körpers aufweist, wobei zumindest ein Paar aneinander angrenzender gebogener Abschnitte in verschiedenen Ebenen liegt, die sich kreuzen, wobei der Mandrin vier gebogene Abschnitte aufweist mit einem an der proximalen Seite angeordneten ersten gebogenen Abschnitt, einem an der distalen Seite des ersten gebogenen Abschnitts angeordneten zweiten gebogenen Abschnitt, einem an der distalen Seite des zweiten gebogenen Abschnitts angeordneten dritten gebogenen Abschnitt und einem an der distalen Seite des dritten gebogenen Abschnitts angeordneten vierten gebogenen Abschnitt, **dadurch gekennzeichnet, dass** die Gesamtlänge des Mandrins zwischen 496 und 744 mm beträgt, wobei der erste gebogene Abschnitt in Richtung nach rechts gebogen ist, wobei die Biegerichtungen des ersten gebogenen Abschnitts und des zweiten gebogenen Abschnitts zueinander entgegengesetzt sind, wobei die Biegerichtungen des zweiten gebogenen Abschnitts und des dritten gebogenen Abschnitts die gleichen sind, wobei die Biegerichtungen des dritten gebogenen Abschnitts und des vierten gebogenen Abschnitts zueinander entgegengesetzt sind, wobei der Krümmungsradius des ersten gebogenen Abschnitts (R1) zwischen 42,7 und 64,0 mm, der Krümmungsradius des zweiten gebogenen Abschnitts (R2) zwischen 46,4 und 69,6 mm, der Krümmungsradius des dritten gebogenen Abschnitts (R3) zwischen 18,3 und 27,4 mm und der Krümmungsradius des vierten gebogenen Abschnitts (R4) zwischen 6,3 und 9,4 mm liegt, wobei ein Schnittwinkel zwischen einer Ebene, in welcher der erste gebogene Abschnitt liegt, und einer Ebene, in welcher der zweite gebogene Abschnitt liegt, 150° ± 20° beträgt bezüglich der Schnittlinie der beiden Ebenen, gemessen im Gegenuhrzeigersinn, wobei die Ebene, in welcher der erste gebogene Abschnitt liegt, auf der proximalen Seite angeordnet und die Ebene, in welcher der zweite gebogene Abschnitt liegt, auf der distalen Seite angeordnet ist, wobei ein Schnittwinkel zwischen der Ebene, in welcher der zweite gebogene Abschnitt liegt, und einer Ebene, in welcher der dritte gebogene Abschnitt liegt, 230° ± 20° beträgt bezüglich der Schnittlinie der beiden Ebenen, gemessen im Gegenuhrzeigersinn, wobei die Ebene, in welcher der zweite gebogene Abschnitt liegt, auf der proximalen Seite angeordnet und die Ebene, in welcher der dritte gebogene Abschnitt liegt, auf der distalen Seite angeordnet ist, wobei ein Schnittwinkel zwischen der Ebene, in welcher der dritte gebogene Abschnitt liegt, und einer Ebene, in welcher der vierte gebogene Abschnitt liegt, 320° ± 20° beträgt bezüglich der Schnittlinie der beiden Ebenen, gemessen im Gegenuhrzeigersinn, wobei die Ebene, in welcher der dritte gebogene Abschnitt liegt, auf der proximalen Seite angeordnet und die Ebene, in welcher der vierte gebogene Abschnitt liegt, auf der distalen Seite angeordnet ist, wobei die Länge des linearen Abschnitts an der distalen Spitze 10 bis 20 mm beträgt, wobei der Schnittwinkel zwischen der Ebene, in welcher der vierte gebogene Abschnitt liegt, und dem linearen Abschnitt an der distalen Spitze zwischen - 20° und + 20° beträgt und der Abstand zwischen der Ebene, in welcher der erste gebogene Abschnitt liegt, und der distalen Spitze zwischen 48,0 und 72,0 mm beträgt.

5. Mandrin, der einen elastischen linearen Körper aufweist, wobei der elastische lineare Körper eine Vielzahl von gebogenen Abschnitten zwischen einer proximalen Seite und einer distalen Seite des elastischen linearen Körpers aufweist, wobei zumindest ein Paar aneinander angrenzender gebogener Abschnitte in verschiedenen Ebenen liegt, die sich kreuzen, wobei der Mandrin drei gebogene Abschnitte aufweist mit einem an der proximalen Seite angeordneten ersten gebogenen Abschnitt, einem an der distalen Seite des ersten gebogenen Abschnitts angeordneten zweiten gebogenen Abschnitt und einem an der distalen Seite des zweiten gebogenen Abschnitts angeordneten dritten Abschnitt, **dadurch gekennzeichnet, dass** die Gesamtlänge des Mandrins zwischen 496 und 774 mm beträgt, wobei der erste gebogene Abschnitt in Richtung nach links gebogen ist, wobei die Biegerichtungen des ersten gebogenen Abschnitts und des zweiten gebogenen Abschnitts die gleichen sind, wobei die Biegerichtungen des zweiten gebogenen Abschnitts und des dritten gebogenen Abschnitts die gleichen sind, wobei der Krümmungsradius des ersten gebogenen Abschnitts (R1) zwischen 141,1 und 211,6 mm, der Krümmungsradius des zweiten gebogenen Abschnitts (R2) zwischen 17,3 und 26,0 mm und der Krümmungsradius des dritten gebogenen Abschnitts (R3) zwischen 4,6 und 6,9 mm liegt, wobei ein Schnittwinkel zwischen einer Ebene, in welcher der erste gebogene Abschnitt liegt, und einer Ebene, in welcher der zweite gebogene Abschnitt liegt, 160° ± 20° beträgt bezüglich der Schnittlinie der beiden Ebenen, gemessen im Gegenuhrzeigersinn, wobei die Ebene, in welcher der erste gebogene Abschnitt liegt, auf der proximalen Seite angeordnet und die Ebene, in welcher der zweite gebogene Abschnitt liegt, auf der distalen Seite angeordnet ist, wobei ein Schnittwinkel zwischen der Ebene, in welcher der zweite gebogene Abschnitt liegt, und einer Ebene, in welcher der dritte gebogene Abschnitt liegt, 160° ± 20° beträgt bezüglich der Schnittlinie der beiden Ebenen, gemessen im Gegenuhrzeigersinn, wobei die Ebene, in welcher der zweite gebogene Abschnitt liegt, auf der proximalen Seite angeordnet und die Ebene, in welcher der dritte gebogene Abschnitt liegt, auf der distalen Seite angeordnet ist, wobei der dritte gebogene Abschnitt die Form eines Vollkreises und einer Hälfte aufweist, wobei die Länge des linearen Abschnitts an der distalen Spitze 2 bis 42 mm beträgt, und wobei die Länge zwischen der distalen Spitze und dem Berührungspunkt des Kreises 14,8 bis 20,4 mm beträgt.

6. Mandrin, der einen elastischen linearen Körper aufweist, wobei der elastische lineare Körper eine Vielzahl von gebogenen Abschnitten zwischen einer proximalen Seite und einer distalen Seite des elastischen linearen Körpers aufweist, wobei zumindest ein Paar aneinander angrenzender gebogener Abschnitte in verschiedenen Ebenen liegt, die sich kreuzen, wobei der Mandrin vier gebogene Abschnitte aufweist mit einem an der proximalen Seite angeordneten ersten gebogenen Abschnitt, einem an der distalen Seite des ersten gebogenen Abschnitts angeordneten zweiten gebogenen Abschnitt, einem an der distalen Seite des zweiten gebogenen Abschnitts angeordneten dritten gebogenen Abschnitt und einem an der distalen Seite des dritten gebogenen Abschnitts angeordneten vierten gebogenen Abschnitt, **dadurch gekennzeichnet, dass** die Gesamtlänge des Mandrins zwischen 496 und 744 mm beträgt, wobei der erste gebogene Abschnitt in Richtung nach rechts gebogen ist, wobei die Biegerichtungen des ersten gebogenen Abschnitts und des zweiten gebogenen Abschnitts die gleichen sind, wobei die Biegerichtungen des zweiten gebogenen Abschnitts und des dritten gebogenen Abschnitts zueinander entgegengesetzt sind, wobei die Biegerichtungen des dritten gebogenen Abschnitts und des vierten gebogenen Abschnitts die gleichen sind, wobei der Krümmungsradius des ersten gebogenen Abschnitts (R1) zwischen 114,7 und 172,0 mm, der Krümmungsradius des zweiten gebogenen Abschnitts (R2) zwischen 27,2 und 40,8 mm, der Krümmungsradius des dritten gebogenen Abschnitts (R3) zwischen 18,1 und 27,2 mm und der Krümmungsradius des vierten gebogenen Abschnitts (R4) zwischen 4,6 und 6,9 mm liegt, wobei ein Schnittwinkel zwischen einer Ebene, in welcher der erste gebogene Abschnitt liegt, und einer Ebene, in welcher der zweite gebogene Abschnitt liegt, 0° ± 20° beträgt bezüglich der Schnittlinie der beiden Ebenen, gemessen im Gegenuhrzeigersinn, wobei die Ebene, in welcher der erste gebogene Abschnitt liegt, auf der proximalen Seite angeordnet und die Ebene, in welcher der zweite gebogene Abschnitt liegt, auf der distalen Seite angeordnet ist, wobei ein Schnittwinkel zwischen der Ebene, in welcher der zweite gebogene Abschnitt liegt, und einer Ebene, in welcher der dritte gebogene Abschnitt liegt, 140° ± 20° beträgt bezüglich der Schnittlinie der beiden Ebenen, gemessen im Gegenuhrzeigersinn, wobei die Ebene, in welcher der zweite gebogene Abschnitt liegt, auf der proximalen Seite angeordnet und die Ebene, in welcher der dritte gebogene Abschnitt liegt, auf der distalen Seite angeordnet ist, wobei ein Schnittwinkel zwischen der Ebene, in welcher der dritte gebogene Abschnitt liegt, und einer Ebene, in welcher der vierte gebogene Abschnitt liegt, 190° ± 20° beträgt bezüglich der Schnittlinie der beiden Ebenen, gemessen im Gegenuhrzeigersinn, wobei die Ebene, in welcher der dritte gebogene Abschnitt liegt, auf der proximalen Seite angeordnet und die Ebene, in weicher der vierte gebogene Abschnitt liegt, auf der distalen Seite angeordnet ist, wobei der vierte gebogene Abschnitt die Form eines Vollkreises und einer Hälfte aufweist, und wobei die Länge zwischen der distalen Spitze und dem Berührungspunkt des Kreises 16,0 bis 24,0 mm beträgt.

7. Mandrin, der einen elastischen linearen Körper aufweist, wobei der elastische lineare Körper eine Vielzahl von gebogenen Abschnitten zwischen einer proximalen Seite und einer distalen Seite des elastischen linearen Körpers aufweist, wobei zumindest ein Paar aneinander angrenzender gebogener Abschnitte in verschiedenen Ebenen liegt, die sich kreuzen, wobei der Mandrin zwei gebogene Abschnitte aufweist mit einem an der proximalen Seite angeordneten ersten gebogenen Abschnitt und einem an der distalen Seite angeordneten zweiten gebogenen Abschnitt, **dadurch gekennzeichnet, dass** die Gesamtlänge des Mandrins zwischen 492 und 738 mm beträgt, wobei der erste gebogene Abschnitt in Richtung nach links gebogen ist, wobei die Biegerichtungen des ersten gebogenen Abschnitts und des zweiten gebogenen Abschnitts die gleichen sind, wobei der Krümmungsradius des ersten gebogenen Abschnitts (R1) zwischen 93,9 und 140,8 mm und der Krümmungsradius des zweiten gebogenen Abschnitts (R2) zwischen 19,9 und 29,8 mm liegt, wobei ein Schnittwinkel zwischen einer Ebene, in welcher der erste gebogene Abschnitt liegt, und einer Ebene, in welcher der zweite gebogene Abschnitt liegt, 150° ± 20° beträgt bezüglich der Schnittlinie der beiden Ebenen, gemessen im Gegenuhrzeigersinn, wobei die Ebene, in welcher der erste gebogene Abschnitt liegt, auf der proximalen Seite angeordnet und die Ebene, in welcher der zweite gebogene Abschnitt liegt, auf der distalen Seite angeordnet ist, wobei die Länge des linearen Abschnitts an der distalen Spitze 5 bis 55 mm beträgt, wobei der Schnittwinkel zwischen der Ebene, in welcher der zweite gebogene Abschnitt liegt, und dem linearen Abschnitt an der distalen Spitze zwischen - 20° und + 20° beträgt und der Abstand zwischen der Ebene, in welcher der erste gebogene Abschnitt liegt, und der distalen Spitze zwischen 20,0 und 30,0 mm beträgt.

8. Mandrin, der einen elastischen linearen Körper aufweist, wobei der elastische lineare Körper eine Vielzahl von gebogenen Abschnitten zwischen einer proximalen Seite und einer distalen Seite des elastischen linearen Körpers aufweist, wobei zumindest ein Paar aneinander angrenzender gebogener Abschnitte in verschiedenen Ebenen liegt, die sich kreuzen, wobei der Mandrin drei gebogene Abschnitte aufweist mit einem an der proximalen Seite angeordneten ersten gebogenen Abschnitt, einem an der distalen Seite des ersten gebogenen Abschnitts angeordneten zweiten gebogenen Abschnitt und einem an der distalen Seite des zweiten gebogenen Abschnitts angeordneten dritten Abschnitt, **dadurch gekennzeichnet, dass** die Gesamtlänge des Mandrins zwischen 496 und 744 mm beträgt, wobei der erste gebogene Abschnitt in Richtung nach rechts gebogen ist, wobei die Biegerichtungen des ersten gebogenen Abschnitts und des zweiten gebogenen Abschnitts die gleichen sind, wobei die Biegerichtungen des zweiten gebogenen Abschnitts und des dritten gebogenen Abschnitts zueinander entgegengesetzt sind, wobei der Krümmungsradius des ersten gebogenen Abschnitts (R1) zwischen 96,0 und 144,0 mm, der Krümmungsradius des zweiten gebogenen Abschnitts (R2) zwischen 36,8 und 55,2 mm und der Krümmungsradius des dritten gebogenen Abschnitts (R3) zwischen 16,8 und 25,2 mm liegt, wobei ein Schnittwinkel zwischen einer Ebene, in welcher der erste gebogene Abschnitt liegt, und einer Ebene, in welcher der zweite gebogene Abschnitt liegt, 0° ± 20° beträgt bezüglich der Schnittlinie der beiden Ebenen, gemessen im Gegenuhrzeigersinn, wobei die Ebene, in welcher der erste gebogene Abschnitt liegt, auf der proximalen Seite angeordnet und die Ebene, in welcher der zweite gebogene Abschnitt liegt, auf der distalen Seite angeordnet ist, wobei ein Schnittwinkel zwischen der Ebene, in welcher der zweite gebogene Abschnitt liegt, und einer Ebene, in weicher der dritte gebogene Abschnitt liegt, 140° ± 20° beträgt bezüglich der Schnittlinie der beiden Ebenen, gemessen im Gegenuhrzeigersinn, wobei die Ebene, in welcher der zweite gebogene Abschnitt liegt, auf der proximalen Seite angeordnet und die Ebene, in welcher der dritte gebogene Abschnitt liegt, auf der distalen Seite angeordnet ist, wobei die Länge des linearen Abschnitts an der distalen Spitze 5 bis 55 mm beträgt, wobei der Schnittwinkel zwischen der Ebene, in welcher der zweite gebogene Abschnitt liegt, und dem linearen Abschnitt an der distalen Spitze zwischen -20° und +20° beträgt, und wobei der Abstand zwischen der Ebene, in welcher der erste gebogene Abschnitt liegt, und der distalen Spitze 28,9 bis 43,4 mm beträgt.

9. Mandrin gemäß irgendeinem der Ansprüche 1 bis 8, wobei der elastische lineare Körper ein Metalldraht mit einem Durchmesser von 0,1 bis 1 mm ist, und wobei ein Griff an dem proximalen Ende des Mandrins angeordnet ist.

10. Mandrin gemäß irgendeinem der Ansprüche 1 bis 9, wobei der Mandrin ein Mandrin zur Anordnung einer Verweilelektrode eines Herzschrittmachers ist.

## Revendications

1. Mandrin comprenant un corps linéaire élastique, le dit corps linéaire élastique comprenant une pluralité de parties courbées entre un côté proximal et un côté distal du corps linéaire élastique, dans lequel au moins une paire de parties courbées adjacentes se trouve dans des plans différents qui se croisent, le dit mandrin comprenant deux parties courbées comprenant une première partie courbée disposée sur le côté proximal et une deuxième partie courbée disposée sur le côté distal, **caractérisé en ce que** la longueur entière du mandrin est comprise entre 442 et 662 mm, la première partie courbée étant courbée à gauche, les directions de courbure de la première partie courbée et de la deuxième partie courbée étant les mêmes, le rayon de courbure de la première partie courbée (R1) étant 80,5 à 120,8 mm, le rayon de courbure de la deuxième partie courbée étant 12,6 à 18,8 mm, l'angle de coupe entre un plan, dans lequel se trouve la première partie courbée, et un plan, dans lequel se trouve la deuxième partie courbée, étant 250° ± 20° par rapport à la ligne d'intersection des deux plans, mesuré dans le sens inverse des aiguilles d'une montre, le plan, dans lequel se trouve la première partie courbée, étant disposé sur le côté proximal, et le plan, dans lequel se trouve la deuxième partie courbée, étant disposé sur le côté distal, la longueur de la partie linéaire à la pointe distale étant comprise entre 5 et 15 mm, l'angle de coupe entre le plan, dans lequel se trouve la deuxième partie courbée, et la partie linéaire à la pointe distale étant compris entre - 20° et + 20° et la distance entre le plan, dans lequel se trouve la première partie courbée, et la pointe distale étant comprise entre 24,4 et 36,6 mm.

2. Mandrin comprenant un corps linéaire élastique, le dit corps linéaire élastique comprenant une pluralité de parties courbées entre un côté proximal et un côté distal du corps linéaire élastique, dans lequel au moins une paire de parties courbées adjacentes se trouve dans des plans différents qui se croisent, le dit mandrin comprenant trois parties courbées comprenant une première partie courbée disposée sur le côté proximal, une deuxième partie courbée disposée sur le côté distal de la première partie courbée et une troisième partie courbée disposée sur le côté distal de la deuxième partie courbée, **caractérisé en ce que** la longueur entière du mandrin est comprise entre 442 et 662 mm, la première partie courbée étant courbée à droite, les directions de courbure de la première partie courbée et de la deuxième partie courbée étant les mêmes, les directions de courbure de la deuxième partie courbée et de la troisième partie courbée étant opposées l'une par rapport à l'autre, le rayon de courbure de la première partie courbée (R1) étant 90,9 à 136,4 mm, le rayon de courbure de la deuxième partie courbée (R2) étant 24,7 à 37,1 mm, le rayon de courbure de la troisième partie courbée (R3) étant 11,5 à 17,3 mm, l'angle de coupe entre un plan, dans lequel se trouve la première partie courbée, et un plan, dans lequel se trouve la deuxième partie courbée, étant 0° ± 20° par rapport à la ligne d'intersection des deux plans, mesuré dans le sens inverse des aiguilles d'une montre, le plan, dans lequel se trouve la première partie courbée, étant disposé sur le côté proximal, et le plan, dans lequel se trouve la deuxième partie courbée, étant disposé sur le côté distal, l'angle de coupe entre un plan, dans lequel se trouve la deuxième partie courbée, et un plan, dans lequel se trouve la troisième partie courbée, étant 230° ± 20° par rapport à la ligne d'intersection des deux plans, mesuré dans le sens inverse des aiguilles d'une montre, le plan, dans lequel se trouve la deuxième partie courbée, étant disposé sur le côté proximal, et le plan, dans lequel se trouve la troisième partie courbée, étant disposé sur le côté distal, la longueur de la partie linéaire à la pointe distale étant comprise entre 5 et 15 mm, l'angle de coupe entre le plan, dans lequel se trouve la troisième partie courbée, et la partie linéaire à la pointe distale étant compris entre - 20° et + 20° et la distance entre le plan, dans lequel se trouve la première partie courbée, et la pointe distale étant comprise entre 18,5 et 27,8 mm.

3. Mandrin comprenant un corps linéaire élastique, le dit corps linéaire élastique comprenant une pluralité de parties courbées entre un côté proximal et un côté distal du corps linéaire élastique, dans lequel au moins une paire de parties courbées adjacentes se trouve dans des plans différents qui se croisent, le dit mandrin comprenant quatre parties courbées comprenant une première partie courbée disposée sur le côté proximal, une deuxième partie courbée disposée sur le côté distal de la première partie courbée, une troisième partie courbée disposée sur le côté distal de la deuxième partie courbée et une quatrième partie courbée disposée sur le côté distai de la troisième partie courbée, **caractérisé en ce que** la longueur entière du mandrin est comprise entre 496 et 744 mm, la première partie courbée étant courbée à gauche, les directions de courbure de la première partie courbée et de la deuxième partie courbée étant les mêmes, les directions de courbure de la deuxième partie courbée et de la troisième partie courbée étant les mêmes, les directions de courbure de la troisième partie courbée et de la quatrième partie courbée étant opposées l'une par rapport à l'autre, le rayon de courbure de la première partie courbée (R1) étant 66,4 à 99,6 mm, le rayon de courbure de la deuxième partie courbée étant 66,7 à 100,0 mm, le rayon de courbure de la troisième partie courbée (R2) étant 17,4 à 26,2 mm, le rayon de courbure de la quatrième partie courbée (R3) étant 6,6 à 9,9 mm, l'angle de coupe entre un plan, dans lequel se trouve la première partie courbée, et un plan, dans lequel se trouve la deuxième partie courbée, étant 0° ± 20° par rapport à la ligne d'intersection des deux plans, mesuré dans le sens inverse des aiguilles d'une montre, le plan, dans lequel se trouve la première partie courbée, étant disposé sur le côté proximal, et le plan, dans lequel se trouve la deuxième partie courbée, étant disposé sur le côté distal, l'angle de coupe entre un plan, dans lequel se trouve la deuxième partie courbée, et un plan, dans lequel se trouve la troisième partie courbée, étant 220° ± 20° par rapport à la ligne d'intersection des deux plans, mesuré dans le sens inverse des aiguilles d'une montre, le plan, dans lequel se trouve la deuxième partie courbée, étant disposé sur le côté proximal, et le plan, dans lequel se trouve la troisième partie courbée, étant disposé sur le côté distal, l'angle de coupe entre un plan, dans lequel se trouve la troisième partie courbée, et un plan, dans lequel se trouve la quatrième partie courbée, étant 330° ± 20° par rapport à la ligne d'intersection des deux plans, mesuré dans le sens inverse des aiguilles d'une montre, le plan, dans lequel se trouve la troisième partie courbée, étant disposé sur le côté proximal, et le plan, dans lequel se trouve la quatrième partie courbée, étant disposé sur le côté distal, la longueur de la partie linéaire à la pointe distale étant comprise entre 10 et 20 mm, l'angle de coupe entre le plan, dans lequel se trouve la quatrième partie courbée, et la partie linéaire à la pointe distale étant compris entre - 20° et + 20° et la distance entre le plan, dans lequel se trouve la première partie courbée, et la pointe distale étant comprise entre 36,4 et 54,6 mm.

4. Mandrin comprenant un corps linéaire élastique, le dit corps linéaire élastique comprenant une pluralité de parties courbées entre un côté proximal et un côté distal du corps linéaire élastique, dans lequel au moins une paire de parties courbées adjacentes se trouve dans des plans différents qui se croisent, le dit mandrin comprenant quatre parties courbées comprenant une première partie courbée disposée sur le côté proximal, une deuxième partie courbée disposée sur le côté distal de la première partie courbée, une troisième partie courbée disposée sur le côté distal de la deuxième partie courbée et une quatrième partie courbée disposée sur le côté distal de la troisième partie courbée, **caractérisé en ce que** la longueur entière du mandrin est comprise entre 496 et 744 mm, la première partie courbée étant courbée à droite, les directions de courbure de la première partie courbée et de la deuxième partie courbée étant opposées l'une par rapport à l'autre, les directions de courbure de la deuxième partie courbée et de la troisième partie courbée étant les mêmes, les directions de courbure de la troisième partie courbée et de la quatrième partie courbée étant opposées l'une par rapport à l'autre, le rayon de courbure de la première partie courbée (R1) étant 42,7 à 64,0 mm, le rayon de courbure de la deuxième partie courbée (R2) étant 46,4 à 69,6 mm, le rayon de courbure de la troisième partie courbée (R3) étant 18,3 à 27,4 mm, le rayon de courbure de la quatrième partie courbée (R4) étant 6,3 à 9,4 mm, l'angle de coupe entre un plan, dans lequel se trouve la première partie courbée, et un plan, dans lequel se trouve la deuxième partie courbée, étant 150° ± 20° par rapport à la ligne d'intersection des deux plans, mesuré dans le sens inverse des aiguilles d'une montre, le plan, dans lequel se trouve la première partie courbée, étant disposé sur le côté proximal, et le plan, dans lequel se trouve la deuxième partie courbée, étant disposé sur le côté distal, l'angle de coupe entre le plan, dans lequel se trouve la deuxième partie courbée, et un plan, dans lequel se trouve la troisième partie courbée, étant 230° ± 20° par rapport à la ligne d'intersection des deux plans, mesuré dans le sens inverse des aiguilles d'une montre, le plan, dans lequel se trouve la deuxième partie courbée, étant disposé sur le côté proximal, et le plan, dans lequel se trouve la troisième partie courbée, étant disposé sur le côté distal, l'angle de coupe entre le plan, dans lequel se trouve la troisième partie courbée, et un plan, dans lequel se trouve la quatrième partie courbée, étant 320° ± 20° par rapport à la ligne d'intersection des deux plans, mesuré dans le sens inverse des aiguilles d'une montre, le plan, dans lequel se trouve la troisième partie courbée, étant disposé sur le côté proximal, et le plan, dans lequel se trouve la quatrième partie courbée, étant disposé sur le côté distal, la longueur de la partie linéaire à la pointe distale étant comprise entre 10 et 20 mm, l'angle de coupe entre le plan, dans lequel se trouve la quatrième partie courbée, et la partie linéaire à la pointe distale étant compris entre - 20° et + 20° et la distance entre le plan, dans lequel se trouve la première partie courbée, et la pointe distale étant comprise entre 48,0 et 72,0 mm.

5. Mandrin comprenant un corps linéaire élastique, le dit corps linéaire élastique comprenant une pluralité de parties courbées entre un côté proximal et un côté distal du corps linéaire élastique, dans lequel au moins une paire de parties courbées adjacentes se trouve dans des plans différents qui se croisent, le dit mandrin comprenant trois parties courbées comprenant une première partie courbée disposée sur le côté proximal, une deuxième partie courbée disposée sur le côté distal de la première partie courbée et une troisième partie courbée disposée sur le côté distal de la deuxième partie courbée, **caractérisé en ce que** la longueur entière du mandrin est comprise entre 496 et 744 mm, la première partie courbée étant courbée à gauche, les directions de courbure de la première partie courbée et de la deuxième partie courbée étant les mêmes, les directions de courbure de la deuxième partie courbée et de la troisième partie courbée étant les mêmes, le rayon de courbure de la première partie courbée (R1) étant 141,1 à 211,6 mm, le rayon de courbure de la deuxième partie courbée (R2) étant 17,3 à 26,0 mm, le rayon de courbure de la troisième partie courbée (R3) étant 4,6 à 6,9 mm, l'angle de coupe entre un plan, dans lequel se trouve la première partie courbée, et un plan, dans lequel se trouve la deuxième partie courbée, étant 160° ± 20° par rapport à la ligne d'intersection des deux plans, mesuré dans le sens inverse des aiguilles d'une montre, le plan, dans lequel se trouve la première partie courbée, étant disposé sur le côté proximal, et le plan, dans lequel se trouve la deuxième partie courbée, étant disposé sur le côté distal, l'angle de coupe entre le plan, dans lequel se trouve la deuxième partie courbée, et un plan, dans lequel se trouve la troisième partie courbée, étant 160° ± 20° par rapport à la ligne d'intersection des deux plans, mesuré dans le sens inverse des aiguilles d'une montre, le plan, dans lequel se trouve la deuxième partie courbée, étant disposé sur le côté proximal, et le plan, dans lequel se trouve la troisième partie courbée, étant disposé sur le côté distal, la troisième partie courbée ayant la forme d'un cercle entier et demi, la longueur de la partie linéaire à la pointe distale étant comprise entre 2 et 42 mm, et la longueur entre la pointe distale et le point de contact du cercle étant comprise entre 14,8 et 20,4 mm.

6. Mandrin comprenant un corps linéaire élastique, le dit corps linéaire élastique comprenant une pluralité de parties courbées entre un côté proximal et un côté distal du corps linéaire élastique, dans lequel au moins une paire de parties courbées adjacentes se trouve dans des plans différents qui se croisent, le dit mandrin comprenant quatre parties courbées comprenant une première partie courbée disposée sur le côté proximal, une deuxième partie courbée disposée sur le côté distal de la première partie courbée, une troisième partie courbée disposée sur le côté distal de la deuxième partie courbée et une quatrième partie courbée disposée sur le côté distal de la troisième partie courbée, **caractérisé en ce que** la longueur entière du mandrin est comprise entre 496 et 744 mm, la première partie courbée étant courbée à droite, les directions de courbure de la première partie courbée et de la deuxième partie courbée étant les mêmes, les directions de courbure de la deuxième partie courbée et de la troisième partie courbée étant opposées l'une par rapport à l'autre, les directions de courbure de la troisième partie courbée et de la quatrième partie courbée étant les mêmes, le rayon de courbure de la première partie courbée (R1) étant 114,7 à 172,0 mm, le rayon de courbure de la deuxième partie courbée (R2) étant 27,2 à 40,8 mm, le rayon de courbure de la troisième partie courbée (R3) étant 18,1 à 27,2 mm, le rayon de courbure de la quatrième partie courbée (R4) étant 4,6 à 6,9 mm, l'angle de coupe entre un plan, dans lequel se trouve la première partie courbée, et un plan, dans lequel se trouve la deuxième partie courbée, étant 0° ± 20° par rapport à la ligne d'intersection des deux plans, mesuré dans le sens inverse des aiguilles d'une montre, le plan, dans lequel se trouve la première partie courbée, étant disposé sur le côté proximal, et le plan, dans lequel se trouve la deuxième partie courbée, étant disposé sur le côté distal, l'angle de coupe entre le plan, dans lequel se trouve la deuxième partie courbée, et un plan, dans lequel se trouve la troisième partie courbée, étant 140° ± 20° par rapport à la ligne d'intersection des deux plans, mesuré dans le sens inverse des aiguilles d'une montre, le plan, dans lequel se trouve la deuxième partie courbée, étant disposé sur le côté proximal, et le plan, dans lequel se trouve la troisième partie courbée, étant disposé sur le côté distal, l'angle de coupe entre le plan, dans lequel se trouve la troisième partie courbée, et un plan, dans lequel se trouve la quatrième partie courbée, étant 190° ± 20° par rapport à la ligne d'intersection des deux plans, mesuré dans le sens inverse des aiguilles d'une montre, le plan, dans lequel se trouve la troisième partie courbée, étant disposé sur le côté proximal, et le plan, dans lequel se trouve la quatrième partie courbée, étant disposé sur le côté distal, la quatrième partie courbée ayant la forme d'un cercle entier et demi, et la longueur entre la pointe distale et le point de contact du cercle étant comprise entre 16,0 et 24,0 mm.

7. Mandrin comprenant un corps linéaire élastique, le dit corps linéaire élastique comprenant une pluralité de parties courbées entre un côté proximal et un côté distal du corps linéaire élastique, dans lequel au moins une paire de parties courbées adjacentes se trouve dans des plans différents qui se croisent, le dit mandrin comprenant deux parties courbées comprenant une première partie courbée disposée sur le côté proximal et une deuxième partie courbée disposée sur le côté distal, **caractérisé en ce que** la longueur entière du mandrin est comprise entre 492 et 738 mm, la première partie courbée étant courbée à gauche, les directions de courbure de la première partie courbée et de la deuxième partie courbée étant les mêmes, le rayon de courbure de la première partie courbée (R1) étant 93,9 à 140,8 mm, le rayon de courbure de la deuxième partie courbée étant 19,9 à 29,8 mm, l'angle de coupe entre un plan, dans lequel se trouve la première partie courbée, et un plan, dans lequel se trouve la deuxième partie courbée, étant 150° ± 20° par rapport à la ligne d'intersection des deux plans, mesuré dans le sens inverse des aiguilles d'une montre, le plan, dans lequel se trouve la première partie courbée, étant disposé sur le côté proximal, et le plan, dans lequel se trouve la deuxième partie courbée, étant disposé sur le côté distal, la longueur de la partie linéaire à la pointe distale étant comprise entre 5 et 55 mm, l'angle de coupe entre le plan, dans lequel se trouve la deuxième partie courbée, et la partie linéaire à la pointe distale étant compris entre - 20° et + 20° et la distance entre le plan, dans lequel se trouve la première partie courbée, et la pointe distale étant comprise entre 20,0 et 30,0 mm.

8. Mandrin comprenant un corps linéaire élastique, le dit corps linéaire élastique comprenant une pluralité de parties courbées entre un côté proximal et un côté distal du corps linéaire élastique, dans lequel au moins une paire de parties courbées adjacentes se trouve dans des plans différents qui se croisent, le dit mandrin comprenant trois parties courbées comprenant une première partie courbée disposée sur le côté proximal, une deuxième partie courbée disposée sur le côté distal de la première partie courbée et une troisième partie courbée disposée sur le côté distal de la deuxième partie courbée, **caractérisé en ce que** la longueur entière du mandrin est comprise entre 496 et 744 mm, la première partie courbée étant courbée à droite, les directions de courbure de la première partie courbée et de la deuxième partie courbée étant les mêmes, les directions de courbure de la deuxième partie courbée et de la troisième partie courbée étant opposées l'une par rapport à l'autre, le rayon de courbure de la première partie courbée (R1) étant 96,0 à 144,0 mm, le rayon de courbure de la deuxième partie courbée (R2) étant 36,8 à 55,2 mm, le rayon de courbure de la troisième partie courbée (R3) étant 16,8 à 25,2 mm, l'angle de coupe entre un plan, dans lequel se trouve la première partie courbée, et un plan, dans lequel se trouve la deuxième partie courbée, étant 0° ± 20° par rapport à la ligne d'intersection des deux plans, mesuré dans le sens inverse des aiguilles d'une montre, le plan, dans lequel se trouve la première partie courbée, étant disposé sur le côté proximal, et le plan, dans lequel se trouve la deuxième partie courbée, étant disposé sur le côté distal, l'angle de coupe entre le plan, dans lequel se trouve la deuxième partie courbée, et un plan, dans lequel se trouve la troisième partie courbée, étant 140° ± 20° par rapport à la ligne d'intersection des deux plans, mesuré dans le sens inverse des aiguilles d'une montre, le plan, dans lequel se trouve la deuxième partie courbée, étant disposé sur le côté proximal, et le plan, dans lequel se trouve la troisième partie courbée, étant disposé sur le côté distal, la longueur de la partie linéaire à la pointe distale étant comprise entre 5 et 55 mm, l'angle de coupe entre le plan, dans lequel se trouve la deuxième partie courbée, et la partie linéaire à la pointe distale étant compris entre - 20° et + 20° et la distance entre le plan, dans lequel se trouve la première partie courbée, et la pointe distale étant comprise entre 28,9 et 43,4 mm.

9. Mandrin selon l'une quelconque des revendications 1 à 8, dans lequel le corps linéaire élastique est un fil métallique ayant un diamètre de 0,1 à 1 mm et une poignée est disposée à l'extrémité proximale du mandrin.

10. Mandrin selon l'une quelconque des revendications 1 à 9, dans lequel le mandrin est un mandrin destiné à placer une électrode à demeure d'un stimulateur cardiaque.
